# EUROPEAN PATENT APPLICATION

(11) **EP 4 730 353 A1**
(43) Date of publication of application: **22.04.2026**
(21) Application number: 25209589.8
(22) Date of filing: 17.10.2025
(51) Int. Cl.: G16H 40/20, G16H 50/20

(54) **SYSTEM AND METHOD FOR OPERATIONAL PRESSURE DETECTION IN A MEDICAL DEVICE ENVIRONMENT**

(30) Priority: 18.10.2024 US 202463708866 P; 30.09.2025 US 202519345154
(71) Applicant: GE Precision Healthcare LLC, Waukesha, WI 53188 (US)
(72) Inventor: FIXLER, Tamas, Waukesha, 53188 (US); HANSEN, Jacqueline, Waukesha, 53188 (US); HOLMES, Clay, Marietta, 30064 (US); NAYLOR, Jennifer, Waukesha, 53188 (US); TREML, Ryan, Waukesha, 53188 (US); BUSH, Bree, Waukesha, 53188 (US); WARRINGTON, Andrew, Bellevue, 98004 (US); BETMAN, Gregory Peter, Chicago, 60661 (US)
(74) Representative: Kilburn & Strode LLP

(57) **Abstract**

A system can comprise a memory that can store computer executable components. The system can further comprise a processor that can execute at least one of the computer executable components. The computer executable components can comprise a data ingestion component that receives operational data associated with a plurality of healthcare entities; a pressure modeling component that calculates real-time pressure scores for the healthcare entities; a forecasting component that applies an artificial intelligence model to forecast pressure scores for the healthcare entities; a scoring component that assigns pressure levels to healthcare entities; an action recommendation component that identifies one or more prescriptive actions for reducing or preventing forecasted pressure; and a user interface component that displays a graphical interface comprising a pressure forecast visualization, a hot spot summary, and one or more selectable actions.

## Description

### CROSS REFERENCE TO RELATED APPLICATIONS

This application claims priority to and the benefit of U.S. Provisional Patent Application No. 63/708,866 filed 10/18/24, the entire contents of which are incorporated by reference herein in their entirety.

### TECHNICAL FIELD

The subject disclosure relates generally to artificial intelligence-based forecasting and decision support systems in healthcare operations management, and more specifically, to systems and methods for predicting resource pressure across hospital departments and recommending prescriptive actions to mitigate operational bottlenecks.

### BACKGROUND

Modern healthcare systems face increasing operational strain as acute care resources become more constrained amidst growing patient volumes and the rising complexity of care delivery. Efficient progression of patients from intake through treatment and discharge depends on the coordinated use of shared resources, including inpatient beds, nurses, imaging modalities, procedural recovery units, transport teams, and ancillary services. Even isolated disruptions in one area can create cascading delays. For example, a patient may be scheduled for imaging but be unable to proceed due to a lack of transport personnel. Another patient may be medically ready for discharge but remain in place due to a bed cleaning backlog or staffing shortfall.

These localized inefficiencies often accumulate into broader system-level effects, including extended wait times, care delays, resource underutilization in some areas, and resource saturation in others. Clinical and administrative leaders frequently rely on manual workflows and fragmented information sources to identify and manage these pressures. Operational coordination is often conducted through recurring team huddles, calls, or shift reports, which may divert clinical staff from patient-facing responsibilities to relay basic status updates such as discharge readiness, transport status, or procedural backlogs.

Some healthcare organizations have adopted digital dashboards or alerting tools, typically within their electronic medical record systems, to monitor aspects of resource utilization or operational status. These tools often display snapshots of current conditions such as bed occupancy, staffing levels, patient throughput, or pending tasks. While helpful for visibility, such tools are generally limited in their ability to anticipate emerging issues or guide coordinated response. They often operate as passive surveillance mechanisms, relying on users to interpret data across multiple departments and formulate mitigation strategies on their own.

In large, multi-departmental or multi-site hospital systems, the interdependencies among departments such as emergency, inpatient, imaging, therapies, and procedural recovery introduce further complexity. Disruptions in one area can propagate through others, making it difficult to identify the root causes of operational pressure or to prioritize interventions. Static displays of data may not provide sufficient context or foresight for effective action. As a result, care delivery can become reactive, fragmented, and vulnerable to preventable delays.

These challenges highlight ongoing limitations in the current approaches to operational awareness and resource coordination in healthcare settings. Improved techniques for anticipating, contextualizing, and responding to system-wide pressure may be desirable.

### SUMMARY

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, systems, methods, and computer program products facilitate forecasting and management of operational pressure in healthcare environments using artificial intelligence, pressure modeling, and prescriptive decision support. These embodiments support proactive operational coordination across departments, units, modalities, or other healthcare entities within a hospital system.

According to one or more embodiments, a system is provided. The system can comprise a non-transitory computer-readable memory that stores computer-executable components. The system can further comprise a processor that executes the computer-executable components. The computer-executable components can include a data ingestion component that receives operational data associated with a plurality of healthcare entities within a hospital system. The computer-executable components can further include a pressure modeling component that calculates real-time pressure scores for the healthcare entities based on department-specific pressure factors and weightings. The computer-executable components can additionally include a forecasting component that applies an artificial intelligence model to forecast pressure scores for the healthcare entities over a future time window. The computer-executable components can also include a scoring component that assigns pressure levels to healthcare entities using proportional or override logic based on critical factors or critical entities. The computer-executable components can further include an action recommendation component that identifies one or more prescriptive actions for reducing or preventing forecasted pressure, each action associated with an impact score, a cost score, and a trigger condition. Additionally, the computer-executable components can include a user interface component that displays a graphical interface comprising a pressure forecast visualization, a hot spot summary, and one or more selectable actions.

According to one or more embodiments, a computer-implemented method is provided. In various embodiments, the method can comprise receiving operational data from a plurality of healthcare entities within a hospital system, calculating pressure scores based on a weighted sum of normalized pressure factor values, and forecasting future pressure scores using an artificial intelligence model trained on historical pressure data. The method can further comprise assigning pressure levels using override logic for critical factors and proportional logic for aggregate entities, identifying prescriptive actions to address forecasted pressure conditions, and displaying a pressure forecast visualization, hot spot summary, and recommended actions via a graphical user interface.

According to one or more embodiments, a computer program product is provided. In various embodiments, the computer program product can comprise a non-transitory computer-readable memory having program instructions embodied therewith. The program instructions can be executable by a processor to cause the processor to receive operational data from a plurality of healthcare entities. The program instructions can be executable to cause the processor to generate real-time pressure scores based on department-specific pressure factor scores and weightings. The program instructions can be executable to cause the processor to forecast future pressure conditions using an artificial intelligence model trained on historical data. The program instructions can be executable to cause the processor to assign pressure levels using override and proportional logic. The program instructions can be executable to cause the processor to identify prescriptive actions with associated trigger conditions, impact scores, and cost scores. The program instructions can be executable to cause the processor to display pressure forecasts, hot spots, and selectable action recommendations within a graphical user interface.

### DESCRIPTION OF THE DRAWINGS

FIG. 1 illustrates a block diagram of an example, non-limiting system that facilitates forecasting and management of operational pressure in healthcare environments, in accordance with one or more embodiments described herein.
FIG. 2 illustrates a flow diagram of an example, non-limiting computer-implemented method that facilitates forecasting and management of operational pressure in healthcare environments, in accordance with one or more embodiments described herein.
FIG. 3 illustrates a flow diagram of an example, non-limiting computer-implemented method that facilitates forecasting and management of operational pressure in healthcare environments, in accordance with one or more embodiments described herein.
FIG. 4 illustrates an example, non-limiting user interface for visualizing pressure forecasts, current operational pressure levels, and prescriptive actions across multiple healthcare facilities within a regional health system, in accordance with one or more embodiments described herein.
FIGS. 5A and 5B collectively illustrate an example, non-limiting set of department-specific pressure layers and corresponding predictive pressure factors, which can be used by a pressure forecasting system to generate department-level pressure scores across a hospital or health system, in accordance with one or more embodiments described herein.
FIG. 6 illustrates an example, non-limiting flow diagram that depicts how operational pressure can be processed and transformed into prescriptive action recommendations, using both real-time and forecasted inputs, in accordance with one or more embodiments described herein.
FIG. 7 illustrates a hierarchical pressure aggregation model that can represent how operational pressure scores can be derived and rolled up from granular pressure factors to higher-level hospital system pressure indicators, in accordance with one or more embodiments described herein.
FIG. 8 illustrates an example, non-limiting hierarchical framework for calculating, aggregating, and interpreting pressure within a healthcare network, in accordance with one or more embodiments described herein.
FIG. 9 illustrates a block diagram of an example, non-limiting operating environment in which one or more embodiments described herein can be facilitated.
FIG. 10 illustrates an example networking environment operable to execute various implementations described herein.

### DETAILED DESCRIPTION

The following detailed description is merely illustrative and is not intended to limit embodiments or application/uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Modern hospital systems face growing operational complexity as they attempt to deliver timely, high-quality care under increasingly constrained conditions. These constraints can include limited inpatient bed availability, variable staffing levels, bottlenecks in diagnostic imaging and procedural recovery areas, and delays in patient transport. As a result, hospitals can experience unpredictable periods of operational pressure, during which demand exceeds available resources. These conditions can cause delays, inefficiencies, and potential risks to patient outcomes.

The concept of pressure in a hospital setting can manifest in various forms. For example, an emergency department can experience excessive boarding times due to a lack of available inpatient beds, even when several patients are ready for discharge. Imaging departments can face rising turnaround times caused by staff shortages or modality-specific downtime. Procedural areas such as PACU or interventional radiology can become saturated when scheduled surgical cases run late or when patients require extended recovery. These localized issues can propagate throughout the hospital and affect departments that are not directly involved in the underlying constraint.

Traditional tools used to manage hospital operations can include passive dashboards, retrospective reports, and manual coordination routines such as huddles or shift calls. While such tools can surface helpful metrics such as occupancy, patient throughput, or staffing assignments, they typically rely on manual interpretation and retrospective decision-making. These tools can lack predictive capabilities and often do not provide guided recommendations for resolution.

The systems and methods described herein can address these limitations by providing a framework for forecasting and managing operational pressure using artificial intelligence, structured scoring logic, and embedded prescriptive guidance. At a high level, the disclosed system can process operational data from healthcare entities within a hospital system, calculate real-time pressure scores, forecast future pressure levels, and recommend one or more actions to reduce or prevent forecasted bottlenecks. The system's functionality can be delivered through a unified interface that enables visualization, interaction, and decision support.

In some embodiments, the system can include a plurality of computer-executable software components stored in a non-transitory memory and executed by a processor. A data ingestion component can receive operational data from departments, units, modalities, or service lines. The data can include information related to patient arrivals, transport requests, imaging order queues, staffing levels, procedural recovery occupancy, and other operational metrics. These data inputs can correspond to department-specific pressure factors, which can serve as inputs to a scoring model.

A pressure modeling component can calculate real-time pressure scores by evaluating the relevant pressure factors for each healthcare entity and combining them into a normalized weighted score. Normalization can be based on statistical transformations such as percentile rankings or standard deviations that contextualize pressure factor values against historical performance. Each pressure factor can be assigned a configurable weight, and the model can output a continuous pressure score, such as a value from zero to one hundred, for each entity.

A forecasting component can apply a trained artificial intelligence model, such as a deep neural network, to forecast future pressure scores over a defined time horizon. The model can be trained on historical hospital data and can account for temporal dynamics, staffing schedules, day-of-week effects, and seasonal variation. Forecasts can be generated hourly or at another configurable interval. The system can maintain separate forecast models per department or use shared models with department-specific feature mappings. In some implementations, an ensemble forecasting approach can be used, combining multiple machine learning models to improve prediction robustness and accuracy.

A scoring component can assign discrete pressure levels to each entity based on the calculated or forecasted pressure scores. Pressure levels can include Normal, Moderate, Elevated, or High. In some embodiments, the scoring component can apply proportional logic, in which the pressure level of a parent entity is derived from the relative proportion of subordinate entities that exceed threshold scores. Additionally, the scoring component can apply override logic, whereby a critical factor or critical entity automatically elevates an entity's pressure level when its score exceeds a configured threshold. An override validation component can be used to track how often overrides are triggered and can alert users or system administrators when thresholds are frequently exceeded, indicating a need to re-tune configuration values or recalibrate models.

An action recommendation component can analyze forecasted or current pressure scores and identify one or more prescriptive actions designed to reduce or avoid pressure conditions. Each action can be associated with a trigger condition, an impact score, and a cost score. The system can include a configurable mapping between pressure drivers and eligible action types. For example, if ICU capacity is constrained, the system can recommend cleaning available beds, initiating patient downgrades, or enabling surge protocols. The impact score can reflect the estimated operational benefit of the action, while the cost score can indicate implementation effort, disruption, or resource use. The action recommendation component can generate a ranked list of actions based on priority level, impact score, cost score, or a composite impact-to-cost ratio. In some cases, action effectiveness can be tracked over time to determine whether recommended interventions correlate with downstream improvements in pressure, enabling feedback-based tuning and performance optimization.

A user interface component can render a graphical interface that displays real-time and forecasted pressure information, hot spot summaries, and recommended actions. The interface can support filtering by department, unit, or modality, and can include visualizations such as bar charts, time-series graphs, and interactive overlays. Forecasted pressure can be displayed using a color-coded gradient, such as green for low pressure and red for high pressure. A hot spot summary can display a limited number of entities currently or forecasted to experience the highest pressure, each accompanied by a generated summary indicating the dominant pressure driver and expected duration. For example, the system can display that CT pressure is elevated due to a high volume of STAT orders and limited scanner availability, expected to persist for six hours.

In some embodiments, a pressure explanation component can provide contextual explanations of forecasted pressure levels. This component can use rule-based logic or model interpretation techniques to generate summaries of contributing factors. For instance, a pressure score of 93 in PACU can be explained by high surgical volume, reduced discharge velocity, and staff absenteeism. These explanations can enhance trust and interpretability for end users and can assist operational leaders in prioritizing intervention points.

The graphical interface can include selectable elements that allow users to drill down into specific actions or contributing factors. For example, selecting a recommendation to expedite transport can open a list of patients who are eligible for reassignment or movement, based on their care progression and destination availability. A recommendation to clean a bed can link to a list of rooms marked as unclean and unassigned. These features can help surface actionable data in context and reduce the need for manual inquiry or cross-department coordination.

In some embodiments, the user interface can include a selectable icon or control that launches a chat-based interface. The chat agent can respond to natural language queries and support scenario exploration. For example, a user can ask, "What happens to procedural recovery if surgical volume increases on Tuesday?" or "Which departments are most likely to hit high pressure tomorrow morning?" The chat agent can reference the forecasting model and underlying operational data to provide reasoned responses, including projected pressure curves, summary statements, or candidate actions. The interface can further support what-if simulation tools that allow users to modify key parameters, such as staff availability or procedure volume, and view the projected impact on future pressure.

The system can also support data refresh and historical persistence. Forecasted pressure scores can be updated at a predetermined interval based on new data ingestion, model retraining triggers, or scheduled update cycles. Historical pressure data can be stored in a persistent data store, such as a time-series database or structured operational log. This historical data can include both raw operational metrics and derived pressure scores at each level of the hierarchy. The stored data can be used for auditing, analytics, trend evaluation, and ongoing model retraining. In some embodiments, an active learning component can be used to identify low-confidence or anomalous predictions and trigger human-in-the-loop reviews, model validation, or targeted retraining to improve model accuracy over time.

To support system-wide optimization across multiple facilities, a multi-facility coordination engine can be included. This component can analyze inter-hospital capacity, staffing, and transfer protocols to recommend load-balancing strategies, such as redirecting low-acuity patients or elective cases to lower-pressure facilities. In regional health systems, this functionality can assist central operations teams in proactively managing system-wide throughput.

An escalation workflow integration component can be used to route high-priority alerts to clinical operations staff, nursing supervisors, or other stakeholders through existing hospital communication channels such as secure paging systems, EMR-integrated messaging, or mobile alert platforms. For example, when pressure in the emergency department is forecasted to reach critical levels within the next four hours, the system can push a structured alert with recommended actions and linked supporting data to appropriate escalation pathways.

A customizable pressure configuration interface can allow administrative users to define, adjust, and test pressure factor models, weights, thresholds, and critical entity definitions. These configurations can be stored per department or per facility, allowing the system to adapt to local operational policies and infrastructure.

To improve accessibility, the system can provide a mobile-optimized dashboard that allows designated users to monitor forecasted pressure, receive alerts, and initiate action workflows from secure mobile devices. The mobile interface can include condensed visualizations, real-time summaries, and single-tap links to core decision support features, enabling responsive coordination even when staff are not stationed at fixed terminals.

By combining operational data ingestion, real-time scoring, predictive forecasting, override-aware escalation, action prioritization, simulation, explanation, multi-hospital coordination, and interactive visualization, the system can support proactive operational management across a hospital or integrated delivery network. The disclosed approach can help care teams and operational leaders anticipate emerging constraints, prioritize interventions, and coordinate decisions that maintain patient flow and resource balance across complex, interdependent departments.

Various embodiments described herein can be used to implement specific improvements in healthcare operations technology. The disclosed systems and methods can provide a practical application for managing hospital resource pressure through artificial intelligence, real-time data processing, hierarchical scoring, and prescriptive decision support. These technical capabilities can be used to address a concrete problem in healthcare operations: the difficulty of anticipating and mitigating dynamic bottlenecks in staffing, throughput, and resource allocation across departments. Such problems cannot be adequately solved using mental reasoning, pen-and-paper methods, or traditional human-coordinated workflows.

The disclosed invention is not directed to an abstract idea, but instead to a specific technical implementation that can transform incoming operational data into structured pressure scores, future pressure forecasts, and actionable interventions. The outputs of this system can be configured to influence real-world actions within the hospital, such as patient transfers, cleaning schedules, staff assignments, and escalation alerts. These operations can be achieved through non-generic computing components that process data in ways not achievable by conventional monitoring tools.

In various embodiments, a system can include computer-executable components that cannot be performed by the human mind. For example, the system can calculate pressure scores by ingesting multiple time-varying inputs, applying configurable weights, and normalizing values using statistical baselines or historical percentiles. The pressure modeling process can take into account both real-time conditions and past trends, allowing scores to be dynamically adjusted based on context. These operations can require computational resources and can operate on data volumes and update frequencies not manageable by manual workflows.

The system can further include a forecasting component that applies artificial intelligence models to project pressure levels over future time intervals. These models can include neural networks trained on large volumes of historical operational data. Forecasting can account for complex nonlinear dependencies between staffing, patient flow, procedural delays, and resource availability. Training and running these models can require hardware acceleration, and the models can be updated or retrained based on ongoing operational feedback. These forecasting functions can generate outputs that are not human-interpretable until processed and visualized through dedicated components.

Pressure levels can be assigned using a scoring component that applies hierarchical logic. The system can propagate scores from subordinate to parent entities using proportional rules, or it can override scores based on critical thresholds. These rules can be configured to reflect hospital-specific operating conditions, and they can change dynamically based on system state. An override validation component can be included to monitor threshold crossing frequency and can prompt reconfiguration or alerting based on operational patterns.

In addition to identifying where pressure is building, the system can generate prescriptive actions to mitigate forecasted strain. The action recommendation component can be used to select interventions based on their expected impact and cost, and can generate ranked lists that help decision-makers prioritize limited resources. These actions can include cleaning beds, initiating patient transport, reallocating staff, opening surge areas, or shifting procedural volumes. Each action can be associated with operational criteria, and the system can link these recommendations to real-time data to assist users in executing them efficiently.

A user interface component can visualize pressure forecasts and recommendations using color-coded charts, filterable dashboards, and selectable action summaries. The interface can also allow users to explore contributing factors, simulate what-if scenarios, or drill down into unit-level data. In some embodiments, the system can include a chat interface that can respond to natural language queries, enabling users to ask questions about forecasted trends or suggested actions. The chat interface can serve as a lightweight decision support layer and can be accessed via desktop or mobile devices.

Additional components can be used to extend the system's technical functionality. A multi-facility coordination engine can be included to support load balancing across hospitals within a network. This engine can evaluate capacity, case mix, and resource distribution across sites and can recommend inter-facility transfers or case deferrals. An escalation workflow integration component can be configured to push alerts into secure messaging systems, enabling time-sensitive recommendations to be routed to appropriate personnel without requiring manual polling.

The system can also include an active learning component that can identify low-confidence forecasts or anomalies in operational behavior and can flag these for manual review or retraining. Historical data, including forecast outputs and actual outcomes, can be stored in structured logs to support auditing, reporting, and performance optimization. Pressure explanations can be generated using interpretable models or heuristic logic to help users understand why pressure is expected to occur and what is driving the score.

The disclosed invention can be applied in a real-world clinical environment and can influence physical processes. For example, a hospital command center can receive an alert that procedural recovery is forecasted to exceed capacity, along with a ranked list of patients who can be discharged or reassigned. A nurse manager can view a pressure dashboard on a mobile device and take immediate action to initiate transport or reallocate staff. These scenarios demonstrate that the invention is not directed to a purely intellectual process or abstract concept but instead operates in a tangible and transformative manner.

By improving how hospital systems interpret, forecast, and respond to operational pressure, the disclosed embodiments can offer technical improvements in system performance, throughput efficiency, and care delivery coordination. These improvements go beyond automation of known manual steps and can materially enhance how resource constraints are managed at scale.

In various embodiments, a computer program product can be configured to forecast and manage operational pressure across a healthcare system. The computer program product can comprise a non-transitory computer-readable medium storing program instructions that, when executed by one or more processors, can facilitate a range of data processing, forecasting, and visualization operations related to healthcare resource utilization, capacity, and system strain.

The program instructions can include logic to receive and process operational data from a plurality of healthcare entities, such as hospital departments, service lines, care units, or transport teams. This operational data can be ingested from real-time data sources or periodically updated feeds and can include metrics such as staffing levels, bed availability, procedure durations, wait times, boarding counts, transfer statuses, and imaging throughput. In some implementations, data ingestion can involve parsing HL7 messages, reading structured database fields, or consuming API endpoints from hospital information systems.

Upon receiving operational data, the computer program product can generate real-time pressure scores for each entity within the healthcare system. Pressure scores can be calculated based on normalized values of department-specific pressure factors, such as net bed availability, staffing delta, imaging scan turnaround time, or patient transport delays. These pressure factor scores can be weighted and combined using configurable coefficients to produce a unified pressure score for each department, unit, or facility. For example, a telemetry unit may have a pressure score derived from 40% staffing delta, 30% bed availability, and 30% patient throughput indicators.

To enable forward-looking operational awareness, the computer program product can include functionality to forecast future pressure conditions using an artificial intelligence model trained on historical operational data. The artificial intelligence model can comprise a deep neural network or other machine learning architecture capable of ingesting time-series data, learning feature interactions, and producing pressure score predictions over various time horizons. For instance, the model can generate pressure forecasts extending from 1 hour to 72 hours in the future, depending on the historical window and update frequency. Historical inputs can include trends in admissions, staffing shifts, discharge rates, order-to-completion times, or other pressure-driving variables.

Once real-time and forecasted pressure scores have been calculated, the computer program product can assign categorical pressure levels to each entity. These pressure levels can be derived using override logic for critical factors and proportional logic for aggregate entities. For example, a department may be automatically classified as "High Pressure" if its boarding count exceeds a configured critical threshold, regardless of the aggregate score. In contrast, a hospital's overall pressure level may be calculated proportionally based on the distribution of pressure scores across its departments and units. This approach can ensure that both localized risks and systemic stressors are properly reflected in the pressure designations.

In response to pressure levels or forecasts, the computer program product can identify prescriptive actions to mitigate current or future operational strain. Each prescriptive action can be defined with an associated trigger condition, an impact score estimating its effect on pressure reduction, and a cost score reflecting operational burden, staffing effort, or resource usage. For example, an action may include opening an overflow unit when medical beds exceed 95% occupancy, or reassigning procedural staff to emergency intake areas during peak boarding periods. Additional prescriptive actions can include reprioritizing imaging queues, activating rapid discharge protocols, delaying non-urgent surgeries, or initiating ICU downgrade workflows to repurpose high-acuity beds.

The computer program product can include a graphical user interface (GUI) that enables users to interactively explore current and forecasted pressure conditions across the healthcare system. The GUI can present pressure information by time horizon, department, hospital, or service line, and can visualize pressure trends using color-coded bar charts, time series graphs, hotspot maps, or tree-structured hierarchies. Users can interact with the GUI to filter by entity type, severity level, or forecast interval and can drill into specific departments or units to understand the contributing factors behind the assigned pressure score. For instance, a user can select a procedural recovery unit and view a ranked breakdown of contributing factors, such as staffing shortages or boarding delays.

In some embodiments, the graphical user interface can support interactive scenario modeling, allowing users to simulate hypothetical interventions using an integrated AI agent. The user can input an operational change, such as increasing ED staffing by three FTEs or delaying scheduled CT scans by two hours, and the system can simulate the projected impact on pressure over time. The AI agent can then return updated forecasts or recommend optimized alternative strategies. This simulation functionality can be used for daily planning, surge preparation, or resource allocation strategy.

The disclosed system architecture can allow the computer program product to scale across multiple facilities and support a wide range of operational scenarios, enabling hospital administrators, clinical leaders, and command center staff to proactively manage care delivery and reduce the risk of operational bottlenecks. The system can operate in real-time or near-real-time and can integrate with hospital dashboards, mobile devices, or alerting platforms to disseminate pressure insights and mitigation strategies across teams.

A hospital system can include one or more individual hospitals, each comprising multiple departments. Departments can be configured based on functional roles, such as Inpatient, Emergency, Imaging, Therapies, Procedural Recovery, or Incoming Transfers. Each department can be structured as a hierarchy of entities, where a department includes one or more sub-departments, and each sub-department can include a set of operational units, known as points of care. For instance, an inpatient department can include sub-departments defined by level of care or service line (e.g., Adult Medicine or Women's Services), and beneath each sub-department, unit-level entities such as telemetry or step-down units can be defined. A hospital can optionally support multiple inpatient departments that are independently scored and managed, such as separate Adult and Pediatric divisions.

Each point-of-care entity can experience operational pressure and contribute to the overall pressure at higher levels of aggregation. In an Emergency Department, the point-of-care entities can include ED bays, pods, or a waiting room. In Imaging departments, points of care can include modality-specific entities such as MRI, CT, Ultrasound, and Echo. Therapies departments can include entities such as Physical Therapy (PT), Occupational Therapy (OT), and Speech-Language Pathology (SLP). Procedural Recovery departments can contain PACU units, Cath Labs, Endoscopy areas, or Interventional Radiology. Incoming Transfers, while not composed of traditional points of care, can be represented as standalone entities due to their operational role at the system or hospital level.

Operational pressure for any given entity can be defined as a numeric score calculated using pressure factor values. Pressure factors are discretely defined variables that represent measurable indicators of operational burden. Each department can be associated with a set of department-specific pressure factors. For example, the Inpatient department may use pressure factors such as net bed availability and staffing delta, whereas the Emergency Department may use pressure factors including the number of boarders, boarding time, the percentage of patients who left without being seen (LWBS), and mean arrival-to-provider times. Imaging departments may rely on scan turnaround time and preliminary read turnaround time, and Procedural Recovery departments may use boarding time, OR holding delays, and projected overnight boarders. These factors can be normalized and aggregated to compute real-time and forecasted pressure scores.

Two primary methods can be used to determine the pressure score for an entity: the calculated method and the proportional method. In the calculated method, a weighted sum of pressure factor values is computed based on department-specific weights, resulting in a normalized pressure score typically scaled between 1 and 100. Each pressure factor can be individually weighted, and weights can be configured to reflect operational priorities. For instance, staffing delta may be assigned less influence than net beds in the inpatient setting, or vice versa. Additionally, entities can be configured to include critical factors. If a critical factor exceeds a defined threshold, it can override the weighted sum and cause the pressure score of the entity to be set directly to that factor's score.

In the proportional method, used most often for higher-level entities such as departments, hospitals, or the entire system, the pressure score for an entity can be determined by aggregating the pressure scores of the child entities immediately beneath it. For example, a hospital pressure score can be derived from the proportional distribution of pressure levels across its departments. Similarly, a department's pressure score can reflect the weighted pressure status of the constituent units or sub-departments. This method allows system-level visualization of emergent hot spots without requiring direct factor evaluation at every level.

To support historical and forecasted pressure modeling, pressure scores can be computed across three temporal windows: historical (past 8 hours), current (now), and forecasted (up to 72 hours in the future). The artificial intelligence model can use historical pressure factors to forecast future values and derive future pressure scores. All pressure scores - whether historical, current, or future - can be generated at an hourly cadence, allowing high-resolution tracking. Entities at every level, including unit, department, hospital, and system, can receive pressure scores either through direct calculation or via proportional inheritance, depending on their role in the model hierarchy.

Pressure levels can be categorized into four bands: Normal, Moderate, Elevated, or High. These categorical labels can be derived from fixed thresholds applied to the numerical pressure score. For example, a pressure score between 0 and 25 may be categorized as Normal, while scores above 75 may be categorized as High. These categorizations allow operational stakeholders to quickly interpret pressure states and prioritize response.

Each pressure score can be linked to one or more prescriptive action recommendations, each of which may include a trigger condition, an impact score representing estimated benefit, and a cost score representing resource utilization. Actions can be recommended to address forecasted or real-time pressure conditions and can include interventions such as opening overflow units, reassigning staff, adjusting imaging schedules, or expediting discharges. Users can interact with these recommendations via a graphical user interface that displays pressure scores, hot spot summaries, and interactive visualizations. The interface can support click-through capabilities to explore specific pressure factors, view associated patient lists, or simulate operational scenarios with alternate configurations or time horizons. An integrated AI agent can assist users in performing scenario analysis by modifying key assumptions and predicting the effect of actions on forecasted pressure.

Overall, the disclosed system and method can support dynamic pressure monitoring and intervention planning at scale across diverse hospital environments, enabling proactive operational management based on intelligent pressure modeling.

It should be appreciated that the figures and description herein provide non-limiting examples of various embodiments and are not necessarily drawn to scale.

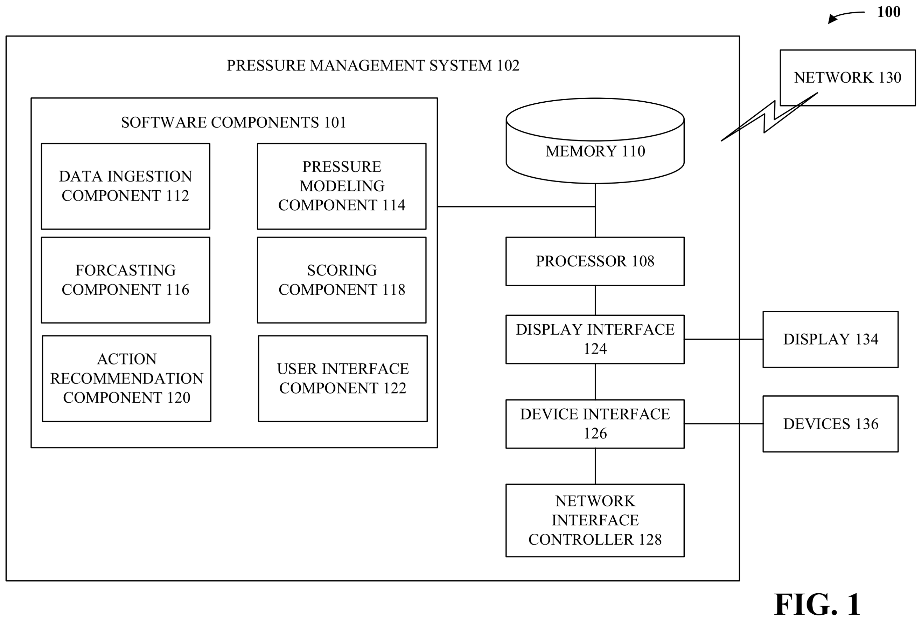

FIG. 1 illustrates a block diagram of an example, non-limiting system 100 that can facilitate real-time forecasting, scoring, and mitigation of operational pressure across a plurality of healthcare entities within a hospital system. In various embodiments, the pressure management system 102 can comprise a processor 108 (e.g., computer processing unit, microprocessor) and a non-transitory computer-readable memory 110 that is operably or operatively or communicatively connected or coupled to the processor 108. The non-transitory computer-readable memory 110 can store computer-executable instructions which, upon execution by the processor 108, can cause the processor 108 or other components of the pressure management system 102 (e.g., data ingestion component 112, pressure modeling component 114, forecasting component 116, scoring component 118, action recommendation component 120, user interface component 122) to perform one or more acts related to pressure modeling, forecasting, scoring, and action recommendation. In various embodiments, the non-transitory computer-readable memory 110 can store computer-executable components (e.g., data ingestion component 112, pressure modeling component 114, forecasting component 116, scoring component 118, action recommendation component 120, user interface component 122), and the processor 108 can execute the computer-executable components.

In various embodiments, pressure management system 102 can comprise a data ingestion component 112. The data ingestion component 112 can receive operational data from a plurality of healthcare entities. These entities can include, for example, inpatient units, emergency departments, imaging departments, operating rooms, intensive care units, procedural recovery units, or ancillary services such as transport or environmental services. The data ingestion component 112 can acquire and process structured or semi-structured operational data related to clinical throughput and resource availability, such as patient counts, bed status, discharge readiness, staff coverage, transport activity, or imaging wait times. Data ingestion component 112 can receive data through interfaces such as HL7 feeds, FHIR APIs, EHR integration points, batch uploads, or secure direct database connections.

The data ingestion component 112 can normalize timestamps across different systems, resolve unit names to standard identifiers, and handle noisy or incomplete data using smoothing or imputation logic. For example, if a data feed is missing a transport completion timestamp, the data ingestion component 112 can infer timing based on patient location changes. Data ingestion component 112 can additionally implement deduplication logic, delta updates, or push-pull syncing protocols to maintain data accuracy and timeliness. The data processed by the data ingestion component 112 can then be passed in real-time to the pressure modeling component 114.

In various embodiments, pressure management system 102 can comprise a pressure modeling component 114. The pressure modeling component 114 can calculate real-time pressure scores for each healthcare entity using department-specific pressure factors and configurable weightings. The pressure modeling component 114 can be configured to treat each entity according to operationally relevant metrics, such as staffed bed availability, patient throughput, procedural backlog, or resource bottlenecks. The pressure modeling component 114 can normalize raw data into percentile scores based on historical distributions for each unit or department, allowing comparisons across time and context.

For example, the pressure modeling component 114 can assign a higher score to a procedural unit with unusually high same-day cancellations and prolonged PACU stays, reflecting elevated procedural flow pressure. The pressure modeling component 114 can also apply exponential decay weights to recent events, giving higher relevance to near-term disruptions while still capturing underlying trends. The output of the pressure modeling component 114 can be a department-level pressure score which can then be routed to the forecasting component 116.

In various embodiments, pressure management system 102 can comprise a forecasting component 116. The forecasting component 116 can apply an artificial intelligence model to project future pressure scores for one or more healthcare entities over a user-defined time window. The forecasting component 116 can use deep learning, gradient-boosted trees, or temporal convolutional models trained on multi-source hospital data including historical pressure factor trends, staffing patterns, admission/discharge data, and ancillary service metrics. The forecasting component 116 can take into account seasonal trends (e.g., flu season), scheduled events (e.g., surgery blocks), and lagged effects between upstream and downstream departments.

For instance, the forecasting component 116 can recognize that a 20% staffing drop in environmental services historically results in bed turnover delays and rising pressure scores in medical-surgical units within two hours. Forecasting component 116 can then generate hourly predictions for up to 48 hours and can flag projected high-pressure intervals. The predictions from the forecasting component 116 can be passed to the scoring component 118 to translate them into actionable levels.

In various embodiments, pressure management system 102 can comprise a scoring component 118. The scoring component 118 can assign pressure levels to healthcare entities using both override and proportional logic. The scoring component 118 can implement predefined escalation rules, such as automatically assigning a "high" pressure level when a critical resource (e.g., ICU nurse staffing) drops below a safe threshold. At the same time, the scoring component 118 can use proportional logic to calculate aggregate service-line or facility-level pressure by combining weighted scores from subordinate departments. For example, scoring component 118 can generate a service-level pressure score for cardiology by aggregating values from cath lab, cardiology inpatient, and telemetry units.

Scoring component 118 can maintain temporal stability using hysteresis thresholds or rolling medians, preventing alert flapping due to transient anomalies. The output of the scoring component 118 can be used by the user interface component 122 to display color-coded levels and can be sent to the action recommendation component 120 for mitigation planning.

In various embodiments, pressure management system 102 can comprise an action recommendation component 120. The action recommendation component 120 can identify prescriptive actions to reduce or prevent forecasted pressure. Each action can be associated with a trigger condition, cost score (e.g., difficulty or resource usage), and impact score (e.g., reduction in predicted pressure). The action recommendation component 120 can include a rules engine, reinforcement learning agent, or hybrid model that selects actions from a curated playbook of hospital interventions. For example, if forecasted pressure is high in the procedural recovery unit, the action recommendation component 120 can suggest reprioritizing elective procedures, reallocating nursing resources, or activating float pool staff.

The action recommendation component 120 can also assess feasibility by querying current capacity. If all transport staff are currently assigned, the action recommendation component 120 can suppress transport-related actions and suggest alternatives such as pre-clearing discharge orders. The action recommendation component 120 can output ranked lists of actions with rationale, estimated time-to-impact, and downstream effects.

In various embodiments, pressure management system 102 can comprise a user interface component 112. The user interface component 122 can display interactive dashboards showing real-time and forecasted pressure, hotspot alerts, and recommended actions. The user interface component 122 can use heatmaps, graphs, scorecards, and drill-down tables to visualize information at the unit, department, or system level. The user interface component 122 can allow filtering by role (e.g., bed manager, transport supervisor), enabling users to view relevant metrics and recommendations.

The user interface component 122 can integrate a scenario simulation tool where users can select an action and see its hypothetical effect on pressure forecasts. The user interface component 122 can also include a natural language assistant that can answer queries like "Why is 3 West at high pressure?" or "What actions are suggested for PACU this afternoon?" All views rendered by the user interface component 122 can be informed by outputs from the scoring component 118, forecasting component 116, and action recommendation component 120.

The processor 108 can interface with a display interface 124, device interface 126, and network interface controller 128. The display interface 124 can communicate with monitors 134, while the device interface 126 can support touchscreens, tablets, or microphones 136 for user input. The network interface controller 128 can connect the pressure management system 102 to a network 130, including secure hospital intranets and cloud data services.

The pressure management system 102 can operate as a modular decision support environment. For example, the data ingestion component 112 can feed updated telemetry to the pressure modeling component 114, whose scores can then flow to the forecasting component 116 for prediction. The predicted scores can then be categorized by the scoring component 118 and fed into the action recommendation component 120, whose recommendations can be reviewed and deployed via the user interface component 122. Each component can work in tight synchronization with the others to ensure responsive, contextual, and anticipatory operational management.

The pressure management system 102 can further comprise a pressure explanation component that can generate interpretability data and plain-language justifications for assigned pressure scores. The pressure explanation component can analyze the input data ingested by the data ingestion component 112 and the resulting pressure scores generated by the pressure modeling component 114 to determine which pressure factors most significantly contributed to a healthcare entity's current or forecasted pressure state. These contributions can be quantified and visualized as ranked drivers, such as "low staffed beds," "transport delays," or "procedural recovery bottlenecks," with associated contribution percentages. For instance, the pressure explanation component can display that "low staffed beds" accounted for 45% of a telemetry unit's pressure score, while "discharge delays" contributed 30%, enabling users to understand the relative operational impact of each driver.

The pressure explanation component can be tightly integrated with the forecasting component 116 and the scoring component 118 to surface which historical trends or predictive features were most influential in elevating the forecasted pressure. For example, if the artificial intelligence model used by the forecasting component 116 identifies a sharp decline in imaging turnaround as a leading indicator of pressure in the emergency department, the pressure explanation component can surface this relationship explicitly, showing users not just what is happening but why it is expected to happen. The pressure explanation component can also coordinate with the user interface component 122 to present this data in interactive formats, such as expandable tooltips or side panels, alongside forecast visualizations.

In some embodiments, the pressure explanation component can further communicate with the action recommendation component 120 to contextualize why specific prescriptive actions were suggested. For example, if the system recommends prioritizing bed cleaning over transport reassignment, the pressure explanation component can explain that the dominant pressure driver was room availability rather than throughput. This coordination can enhance user trust in the system by improving transparency and supporting informed decision-making. The pressure explanation component can therefore serve as a bridge between complex AI-driven computations and human operational intuition, ensuring that users are not only alerted to pressure risks but are also equipped to understand and respond to them effectively.

The pressure management system 102 can further comprise an override validation component that can track, log, and audit all manual overrides made by users to pressure scores, escalation levels, recommended actions, or forecasted outputs. Manual overrides can occur when users with appropriate permissions adjust the system's outputs based on situational awareness, policy exceptions, or emergent factors not captured in real-time data. For example, a nursing supervisor may downgrade the pressure level of a unit after reassigning staff, even if the current staffing metrics have not yet updated in the source system. The override validation component can capture such events and maintain a detailed record of each override action.

The override validation component can store structured metadata for each override, including a unique user identifier, role or credential level, timestamp of the override, the specific field or value changed, the original system-generated value, the manually entered value, a selected justification code or free-text reason, and the downstream effects such as changes in the ranked list of recommended actions. This metadata can be stored in a secure, queryable log that allows authorized administrators to review override histories and evaluate their frequency, appropriateness, and impact on operational decision-making.

The override validation component can interface with the user interface component 122 to present real-time alerts or visual indicators when an override is active on a particular entity. It can also coordinate with the scoring component 118 and action recommendation component 120 to ensure that manual changes are consistently reflected in all dependent outputs. For example, if a user overrides the pressure level for a procedural unit from "elevated" to "normal," the override validation component can trigger a recalculation of downstream recommendations to ensure alignment with the new pressure level. In some embodiments, the override validation component can also generate reports for compliance, audit, or governance workflows and support traceability across system updates, making it a foundational tool for ensuring transparency, accountability, and trust in a semi-automated decision support environment.

The pressure management system 102 can further comprise a facility coordination component that can enable synchronized pressure modeling, forecasting, and mitigation across multiple hospitals, campuses, or affiliated sites within a healthcare system. The facility coordination component can ingest and aggregate operational data from each participating facility using secure network connections, standardized data interfaces, or cloud-based integration frameworks. This aggregation can allow the system to compute and compare pressure scores at the facility level, detect system-wide bottlenecks, and provide a consolidated regional operations dashboard.

The facility coordination component can produce cross-facility visualizations that highlight where pressure is accumulating or easing, enabling enterprise-level leadership to identify which sites are at risk and which have spare capacity. For example, the facility coordination component can display that Hospital A is nearing full occupancy in its telemetry unit while Hospital B has six staffed beds available, prompting transfer planning. The component can generate a comparative table of pressure scores by service line (e.g., cardiology, surgery, imaging) across facilities, allowing centralized decision-makers to direct resource rebalancing strategies.

The facility coordination component can interface with the scoring component 118 to propagate unit-level pressure data upward into facility-wide rollups and then aggregate those into multi-site analytics. It can also interface with the action recommendation component 120 to support enterprise-level interventions, such as reassigning float staff across locations or activating system-wide surge protocols. Additionally, the facility coordination component can support policy-based controls, such as prioritizing patient transfers based on acuity, specialty alignment, or transportation availability.

In some embodiments, the facility coordination component can further include alerting mechanisms that notify regional command centers when certain thresholds are exceeded across facilities. It can support scenario planning across sites by coordinating with the simulation and scenario modeling component, enabling leadership to explore how redirecting procedural volume from one hospital to another might relieve pressure while maintaining care continuity. By providing a centralized operational view and enabling data-driven redistribution of resources, the facility coordination component can strengthen cross-site collaboration and improve resilience across complex health systems.

The pressure management system 102 can further comprise an escalation workflow component that can generate, initiate, and track multi-step operational response plans based on detected or forecasted high-pressure conditions. The escalation workflow component can be configured with rule-based triggers or threshold-based logic that automatically activates predefined escalation sequences when pressure scores or levels exceed specific values. For example, if the emergency department is forecasted to reach a "high" pressure level within the next two hours, the escalation workflow component can initiate a coordinated series of tasks such as notifying the bed management team, activating float pool staff, reprioritizing discharge workflows, or expediting environmental services to clean and prepare additional rooms.

The escalation workflow component can interface with the scoring component 118 and forecasting component 116 to continuously monitor for qualifying conditions, and can work in tandem with the action recommendation component 120 to determine which interventions should be grouped into a structured plan. Each step in the escalation plan can be associated with a designated role, expected completion time, and communication protocol (e.g., page, email, task queue). For instance, upon activation, the escalation workflow component can automatically page the charge nurse to evaluate admission bottlenecks, reallocate staff via the staffing coordinator, and initiate an alert to transport services to expedite patient movement.

To support accountability and transparency, the escalation workflow component can also track task status, user acknowledgments, timestamps, and downstream results. Each step can be marked as pending, in progress, or complete, and exceptions or delays can trigger alerts or secondary workflows. In some embodiments, the escalation workflow component can visualize active workflows in the user interface component 122, allowing users to view the current escalation plan, monitor progress, and modify steps as conditions evolve.

The escalation workflow component can also support reusable templates and scenario-specific customization. Hospitals can define tiered response plans (e.g., for ED crowding, PACU overflow, or ICU saturation) that are automatically tailored based on contextual data. By providing structure, automation, and traceability to operational responses, the escalation workflow component can help hospital systems respond to pressure conditions with speed, coordination, and clarity.

The pressure management system 102 can further comprise a simulation and scenario modeling component that can enable interactive what-if analysis to support proactive decision-making. The simulation and scenario modeling component can allow users to explore the operational impact of hypothetical interventions or changes in resource availability before implementing them in the real environment. For example, a user may use the simulation and scenario modeling component to test whether initiating early discharges for two patients in a high-pressure post-anesthesia care unit (PACU) would meaningfully reduce projected pressure over the next four hours. Similarly, the user can simulate the effects of reassigning transport staff from general medicine to the emergency department to evaluate whether such an action would alleviate anticipated delays in patient movement.

The simulation and scenario modeling component can be integrated with the forecasting component 116 and scoring component 118 to recalculate future pressure scores and pressure levels based on the user-defined adjustments. By injecting simulated changes into the modeled data pipeline - such as artificial discharges, staffing increases, or bed reallocations - the simulation and scenario modeling component can generate revised forecasts that reflect the altered operational conditions. These revised projections can help users evaluate the effectiveness, urgency, and trade-offs of proposed actions under varying assumptions or constraints.

The simulation and scenario modeling component can further interact with the user interface component 122 to present results in a visually intuitive format. Simulated scenarios can be rendered using color-coded graphs, comparative dashboards, or interactive overlays that highlight differences between the baseline forecast and the simulated outcome. Users can run multiple scenarios in parallel, save frequently used configurations, or export insights to operational planning tools. This capability can empower stakeholders to make informed decisions under uncertainty, reduce trial-and-error interventions, and prioritize actions that offer the highest projected impact on relieving operational pressure. he pressure management system 102 can further comprise a mobile interface component that can deliver real-time operational awareness and decision support to clinicians, administrators, and staff via mobile devices such as smartphones and tablets. The mobile interface component can push live pressure alerts, hot spot summaries, and recommended prescriptive actions directly to authorized users, allowing them to monitor evolving conditions and respond to urgent situations while on the move. The mobile interface component can present a streamlined version of the graphical user interface provided by the user interface component 122, optimized for smaller screens and touch-based interaction.

The mobile interface component can allow users to quickly view department-level pressure scores, escalation statuses, and active mitigation workflows, and can support role-specific customization of content. For example, a charge nurse may receive a push notification indicating that their unit has entered a high-pressure state due to delayed discharges and may be prompted to review and approve a proposed intervention such as contacting environmental services. The mobile interface component can allow users to acknowledge, accept, escalate, or defer recommended actions with a single tap, reducing the time between recommendation and execution.

The mobile interface component can also include secure communication features such as role-based access control, encrypted messaging, and integration with hospital paging systems or electronic health record (EHR) mobile apps. By enabling clinicians and operational leaders to engage with the pressure management system 102 from any location within the hospital or across sites, the mobile interface component can improve responsiveness, reduce coordination delays, and ensure that critical pressure mitigation workflows are not bottlenecked by desktop-based access limitations.

The pressure management system 102 can further comprise an ensemble modeling component that can improve the robustness, reliability, and accuracy of pressure forecasts by aggregating outputs from multiple predictive models. The ensemble modeling component can receive intermediate pressure predictions from different model architectures - such as recurrent neural networks, gradient-boosted trees, or attention-based temporal encoders - and can combine them using a dynamic weighting strategy to produce a consensus forecast. These weights can be based on factors such as department type, historical model accuracy for a given unit, temporal forecasting window (e.g., near-term vs. long-range), or operational context.

For example, the ensemble modeling component can prioritize a high-frequency, short-horizon model for emergency department forecasts due to their rapid variation, while favoring a seasonally adjusted trend model for procedural areas with predictable daily volumes. The ensemble modeling component can maintain a performance registry that tracks which models have historically performed best under similar conditions and use that registry to inform real-time weighting logic. In this way, the ensemble modeling component can adaptively shift emphasis across constituent models to respond to evolving data patterns, anomalies, or special events such as hospital system downtimes or holiday surges.

In addition, the ensemble modeling component can serve as a safeguard against model drift or outlier sensitivity. By distributing predictive responsibility across diverse models, the ensemble modeling component can mitigate the effects of data quality issues or localized training biases. This can be especially valuable in complex hospital systems where operational dynamics differ significantly across departments or facilities. The output of the ensemble modeling component can be provided to the forecasting component 116 or used directly by the scoring component 118 to assign future pressure levels. When integrated with the broader pressure management system 102, the ensemble modeling component can support continuous, adaptive learning and maintain high-fidelity forecasts that drive timely and appropriate interventions.

The pressure management system 102 can further comprise an active learning component that can enhance the overall intelligence and adaptability of forecasting models by incorporating human feedback into the model lifecycle. The active learning component can analyze the confidence scores associated with predicted pressure outputs from the forecasting component 116 and identify instances where predictions fall below a configurable confidence threshold. These low-confidence predictions can then be flagged for human review, such as by clinical operations experts, who can provide validation or correction inputs. This process can ensure that edge cases, anomalies, or novel situations are appropriately handled without requiring full dataset retraining.

The active learning component can operate in a continuous loop, where newly labeled examples, validated or corrected by experts, are periodically batched and used to incrementally retrain the underlying AI models. The retraining process can focus only on the most uncertain or informative examples, thereby improving model performance with minimal data labeling overhead. For example, if the system struggles to predict pressure in a newly launched procedural unit with atypical workflows, the active learning component can prioritize that department's data for review and targeted model improvement.

In some embodiments, the active learning component can employ uncertainty sampling techniques, diversity sampling, or Bayesian dropout methods to assess which pressure predictions are most uncertain or influential. The active learning component can also track concept drift and adaptively adjust which data points are surfaced for human review based on shifts in hospital operations or policy. The outputs of the active learning component can feed directly into the forecasting component 116 and the ensemble modeling component to maintain accuracy over time.

The active learning component can additionally provide audit logs of all flagged cases, user inputs, and retraining events, enabling governance and traceability of model evolution. When integrated into the broader pressure management system 102, the active learning component can support continuous learning while preserving clinician oversight and contextual judgment.

The pressure management system 102 can further comprise an audit trail component that can log all data inputs, pressure calculations, forecasts, action recommendations, user interactions, and manual overrides in a secure and queryable format. The audit trail component can assign unique identifiers and timestamps to every data element or user event, thereby ensuring full traceability and data provenance throughout the pressure management workflow. This functionality can be particularly important for regulatory compliance, operational reviews, and post-event analysis.

In one example, if a patient transfer was delayed due to an overridden pressure recommendation, the audit trail component can document when the override was made, by whom, why, and how the delay impacted downstream operational metrics. These logs can be filtered and queried by department, user, action type, or time window, enabling retrospective investigations and compliance auditing.

The audit trail component can also support audit exports, data visualizations, and report generation tools for hospital leadership, quality improvement teams, or external regulators. These outputs can help identify systemic bottlenecks, evaluate policy adherence, or assess the frequency and rationale of manual interventions. Integration with the override validation component can further strengthen audit capabilities by linking system-generated recommendations to real-world user behavior.

The audit trail component can be designed with secure storage, access controls, and encryption to meet hospital data governance and HIPAA requirements. When integrated into the pressure management system 102, the audit trail component can promote accountability, reproducibility, and trust in system-guided decisions.

The pressure management system 102 can further comprise an alert prioritization component that can intelligently filter, rank, and route pressure-related alerts based on user-specific context and operational urgency. The alert prioritization component can assess a variety of factors, such as the user's role, shift timing, prior alert history, and departmental responsibility, to determine which alerts are most relevant or actionable for that user. For example, a transport coordinator nearing the end of their shift may receive only critical alerts affecting the next 30 minutes, while a night-shift supervisor may receive a broader overview of predicted pressure for the next eight hours.

The alert prioritization component can use scoring algorithms that take into account both alert severity and user availability or workload. The alert prioritization component can also suppress or batch lower-priority alerts to reduce cognitive overload and alarm fatigue. Alerts can be color-coded, grouped by theme, or aggregated by unit, allowing users to quickly triage and focus attention where needed. The alert prioritization component can also integrate user feedback to refine its ranking logic over time.

The alert prioritization component can work in conjunction with the user interface component 122 to control how and when alerts are displayed and can be especially useful in environments with limited staffing or high operational complexity. The alert prioritization component can also integrate with the escalation workflow component to ensure timely handoff of unresolved alerts between shifts. When combined with other components of the pressure management system 102, the alert prioritization component can improve signal-to-noise ratio and enhance the efficiency of clinical and operational decision-making.

The pressure management system 102 can further comprise a capacity-based recommendation filter that can evaluate the real-time feasibility of prescriptive actions generated by the action recommendation component 120. Before surfacing a suggested intervention to the user, the capacity-based recommendation filter can assess whether the required resources, such as staff, beds, equipment, or transport availability, are currently in place to support execution of that recommendation. If not, the capacity-based recommendation filter can suppress the recommendation or replace it with a more viable alternative.

For instance, if the action recommendation component 120 suggests reassigning environmental services staff to expedite bed turnover, but the capacity-based recommendation filter detects that all environmental staff are currently occupied or below minimum thresholds, that recommendation can be deprioritized or blocked. Instead, the system may suggest deferring elective admissions, activating float resources, or reprioritizing discharge order entry. This capacity-aware logic can help reduce wasted effort, improve user trust in recommendations, and ensure that proposed actions align with real-world constraints.

The capacity-based recommendation filter can continuously monitor operational data streams ingested by the data ingestion component 112 to assess the availability of critical resources. The capacity-based recommendation filter can also reference predefined business rules, such as staffing minimums or regulatory limits, to validate feasibility. Integration with the user interface component 122 can allow users to understand why certain recommendations are being suppressed or modified.

When used in tandem with the action recommendation component 120, the capacity-based recommendation filter can create a closed-loop recommendation system that adapts not only to pressure forecasts but also to the underlying operational context. This capability can be critical for high-reliability hospital environments where poor recommendations can lead to confusion, alert fatigue, or unintended consequences.

In combination, the components of the pressure management system 102 can function as an integrated, real-time decision support platform capable of anticipating, interpreting, and responding to operational pressure across diverse healthcare environments. By continuously ingesting dynamic hospital data, applying advanced modeling and forecasting techniques, and recommending context-aware actions with traceable justification and feasibility validation, the system can enable healthcare organizations to proactively manage capacity constraints, mitigate bottlenecks, and maintain care quality under evolving conditions. The modular nature of the system allows for extensibility, scalability, and integration with existing hospital workflows, while the rich interpretability, override accountability, and simulation capabilities ensure that human decision-makers remain in control. Together, these features can support a shift from reactive, fragmented operations to coordinated, anticipatory pressure management.

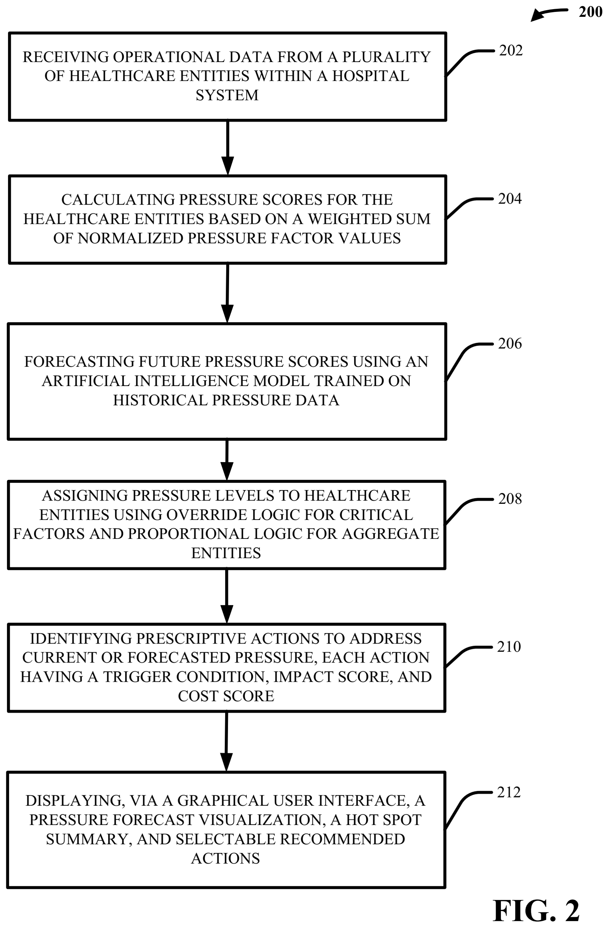

FIG. 2 illustrates a flow diagram of an example, non-limiting computer-implemented method 200 that can facilitate forecasting and managing operational pressure across a plurality of healthcare entities within a hospital system, in accordance with one or more embodiments described herein.

At act 202, the method 200 can include receiving, by a system (e.g., via data ingestion component 112) operatively coupled to a processor (e.g., processor 108), operational data from a plurality of healthcare entities within a hospital system. These healthcare entities can include inpatient units, emergency departments, imaging departments, operating rooms, intensive care units, procedural recovery areas, and ancillary services such as transport, housekeeping, or laboratory departments. The operational data can be received from various sources and interfaces, including real-time HL7 feeds, FHIR-based APIs, EHR data extracts, telemetry sensors, or secure batch uploads. The data ingestion component 112 can process both structured and semi-structured data and can include logic to normalize timestamps, map unit names to standard identifiers, filter outliers, and resolve incomplete records using interpolation or imputation. For example, if a transport completion timestamp is missing for a patient, the system can infer timing based on observed location changes.

At act 204, the method 200 can include calculating, by the system (e.g., via pressure modeling component 114), real-time pressure scores for the healthcare entities based on a weighted sum of normalized pressure factor values. Each healthcare entity can be associated with department-specific pressure factors that reflect key operational attributes relevant to its function. These factors can include staffed bed availability, admission backlog, discharge readiness, environmental services availability, throughput delays, or surgical schedule density. The pressure modeling component 114 can convert raw values into normalized percentiles or z-scores based on historical distributions for each department, enabling cross-unit comparisons. The component can then apply configurable weightings to combine these normalized scores into a real-time pressure score. For example, a procedural unit may have higher weighting on recovery delays, while an emergency department may emphasize waiting room length and diagnostic turnaround times. The output of act 204 can be a pressure score that represents the degree of operational strain experienced by a given entity at a given time.

At act 206, the method 200 can include forecasting, by the system (e.g., via forecasting component 116), future pressure scores for the healthcare entities using an artificial intelligence model trained on historical pressure data. The artificial intelligence model can comprise one or more neural networks, temporal convolutional models, or tree-based regressors trained on multi-dimensional time series data derived from prior operational conditions. The forecasting component 116 can be configured to learn both intra-department and cross-department dependencies over time. For instance, it can recognize that delays in radiology often lead to downstream bottlenecks in emergency care, or that decreased environmental services staffing is predictive of delayed room turnover and rising inpatient pressure. The model can generate pressure predictions at fixed intervals (e.g., every hour) across a defined forecast window (e.g., next 24 or 48 hours). These forecasts can serve as early warnings of anticipated congestion and can support proactive mitigation strategies.

At act 208, the method 200 can include assigning, by the system (e.g., via scoring component 118), pressure levels to the healthcare entities using override logic for critical factors and proportional logic for aggregate entities. The scoring component 118 can translate raw or forecasted pressure scores into discrete levels such as "normal," "moderate," "elevated," or "high." Override logic can be applied when a specific critical factor, such as minimum staffing threshold or ICU occupancy, exceeds a safety limit, resulting in an automatic escalation regardless of overall score. In parallel, proportional logic can be used to compute aggregate pressure levels by weighting and combining the scores of subordinate units or services. For example, if telemetry, cardiac recovery, and cath lab all report high pressure scores, the system can assign a high-level pressure score to the cardiology service line. The scoring component 118 can apply smoothing techniques such as rolling medians to reduce noise and prevent alert fatigue from transient fluctuations.

At act 210, the method 200 can include identifying, by the system (e.g., via action recommendation component 120), one or more prescriptive actions to address current or forecasted pressure. Each prescriptive action can be associated with a specific trigger condition (e.g., pressure level exceeding "elevated"), an estimated impact score representing the anticipated reduction in pressure, and a cost score representing the operational difficulty or resource usage associated with that action. The action recommendation component 120 can retrieve candidate actions from a predefined playbook or dynamically generate recommendations using historical intervention-outcome pairs. For instance, if the procedural recovery unit is predicted to experience high pressure in four hours, the system can recommend early discharges, reassignment of float staff, or procedural rescheduling. Each proposed action can be ranked by priority, efficiency (e.g., impact-to-cost ratio), and feasibility, and can be subject to validation through the capacity-based recommendation filter.

At act 212, the method 200 can include displaying, by the system (e.g., via user interface component 122), a graphical user interface that includes a pressure forecast visualization, a hot spot summary, and one or more selectable recommended actions. The graphical interface can present historical trends and predicted future pressure values using line graphs, heatmaps, or color-coded indicators. Hot spot summaries can list the most critical departments or units, along with the key pressure drivers identified by the pressure explanation component. The selectable recommended actions can be displayed as clickable cards or dropdown options, each annotated with estimated impact, urgency, and expected time to benefit. Users can interact with the interface to explore what-if scenarios, adjust timeframes, or approve suggested interventions. The graphical user interface can be deployed on desktop dashboards or mobile devices and can be tailored to different user roles such as operations managers, nursing supervisors, or hospitalists. By aggregating, contextualizing, and surfacing actionable information, act 212 can support timely, informed decisions that improve operational resilience.

Collectively, the acts of method 200 can enable a data-driven, anticipatory framework for hospital capacity management and real-time operational decision support. The method can be implemented using a combination of rule-based logic, machine learning, and human-in-the-loop workflows, and can be deployed in a modular fashion to support integration with existing hospital IT systems.

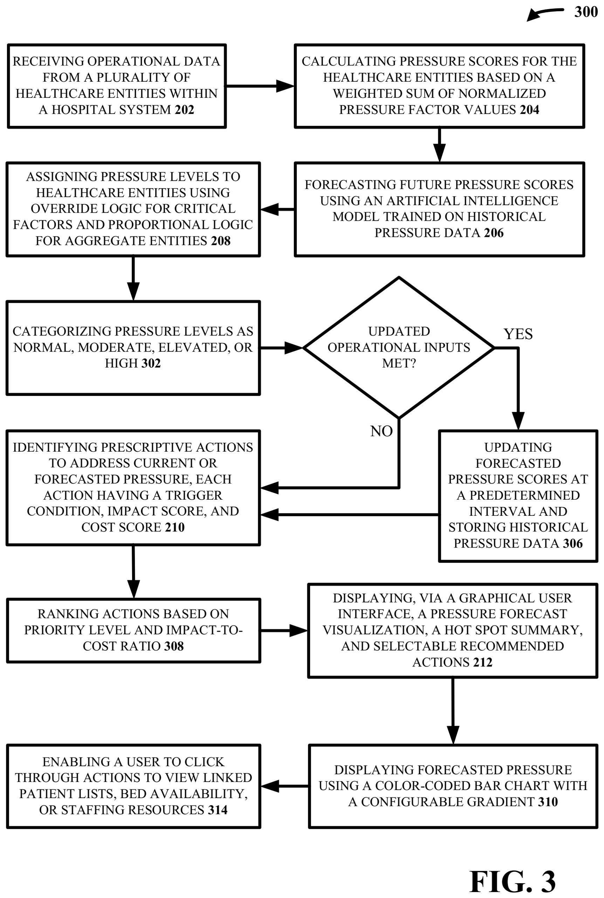

Next, FIG. 3 illustrates an example, non-limiting method 300 that can facilitate forecasting and management of operational pressure in a healthcare system. The method can begin by receiving operational data from a plurality of healthcare entities within a hospital system, as shown in block 202. This data can include real-time and historical information related to staffing levels, bed availability, patient throughput, procedural schedules, discharge activity, imaging turnaround times, and other operational metrics. The received data can be preprocessed, standardized, and validated to ensure consistency and accuracy across healthcare entities that may operate on different information systems or reporting intervals.

At block 204, the method can include calculating pressure scores for each healthcare entity based on a weighted sum of normalized pressure factor values. These pressure factors can be tailored to the specific operational characteristics of each entity, and the normalization process can include percentile ranking, z-score conversion, or historical distribution fitting to enable fair comparison across time periods and departments. The weighting scheme can reflect configurable priorities or empirically derived impact weights, allowing pressure scores to represent a composite view of current operational burden.

At block 206, the method can forecast future pressure scores using an artificial intelligence model trained on historical pressure data. The model can learn temporal patterns, correlations between operational variables, and cascading effects between departments to predict future states under different conditions. Forecasts can be generated at hourly, shift-level, or daily intervals and can include confidence scores or predictive intervals to support downstream decision-making.

Block 208 involves assigning pressure levels to healthcare entities using both override logic and proportional logic. Override logic can trigger when a critical operational factor exceeds a predetermined threshold, regardless of the overall pressure score. For instance, if ICU staffing falls below a safety minimum, the method can automatically assign a high pressure level. Proportional logic can aggregate pressure scores from sub-entities, such as hospital units or procedural areas, to calculate composite scores and assign facility-level pressure levels.

At block 302, the assigned pressure levels can be categorized into discrete labels such as Normal, Moderate, Elevated, or High. These categories can be used to standardize reporting, trigger alerts, or drive user interface color coding. Categorization thresholds can be static or dynamically adjusted based on recent trends or seasonal norms. This step ensures that end users are presented with intuitive summaries that support rapid triage and intervention.

The method can then proceed to a decision point where updated operational inputs are assessed. If the input data has been updated or if the system is operating on a scheduled refresh interval, the method can proceed to block 306. At block 306, the method can update forecasted pressure scores using the most recent operational data and can store these scores as part of the historical dataset. This archival process can support model retraining, audit trails, and longitudinal performance monitoring.

If operational inputs have not been updated, the method can continue using the most recent forecasted data for downstream analysis. At block 210, the method can identify prescriptive actions that may reduce or prevent current or forecasted operational pressure. Each action can be associated with a defined trigger condition, a quantitative impact score representing expected pressure reduction, and a cost score representing resource utilization, implementation effort, or workflow disruption.

At block 308, the method can rank candidate actions based on one or more prioritization metrics. These metrics can include absolute impact, cost-effectiveness (e.g., impact-to-cost ratio), urgency, resource availability, and strategic alignment. Ranked actions can enable users to focus on high-leverage interventions that offer meaningful pressure relief with manageable resource investments.

At block 212, the method can display, via a graphical user interface, a pressure forecast visualization, a hot spot summary, and a set of selectable recommended actions. The graphical user interface can include dynamic charts, interactive maps, alert banners, and control elements to facilitate exploration and decision-making. Forecasts can be visualized over time to show projected pressure trajectories, and hot spot summaries can identify entities at risk of entering elevated or high-pressure states.

At block 310, the method can display forecasted pressure using a color-coded bar chart with a configurable gradient. This visualization technique can translate pressure categories into intuitive visual representations using color schemes such as green to red or blue to orange. The gradient scale can be configured to match institutional preferences, pressure score distributions, or accessibility needs. The visualization can help stakeholders quickly assess the severity and location of pressure across departments or time windows.

At block 314, the method can enable users to click through displayed actions or alerts to access linked operational data such as patient lists, bed availability reports, staffing dashboards, or intervention audit logs. This click-through functionality can provide actionable context to support execution, validation, or escalation of recommended actions. For example, a user may click on a suggested discharge prioritization action to review a list of patients medically ready for discharge, along with their anticipated discharge barriers and responsible care teams.

Collectively, the method steps of FIG. 3 can enable a comprehensive and continuously adaptive approach to operational pressure management in healthcare settings.

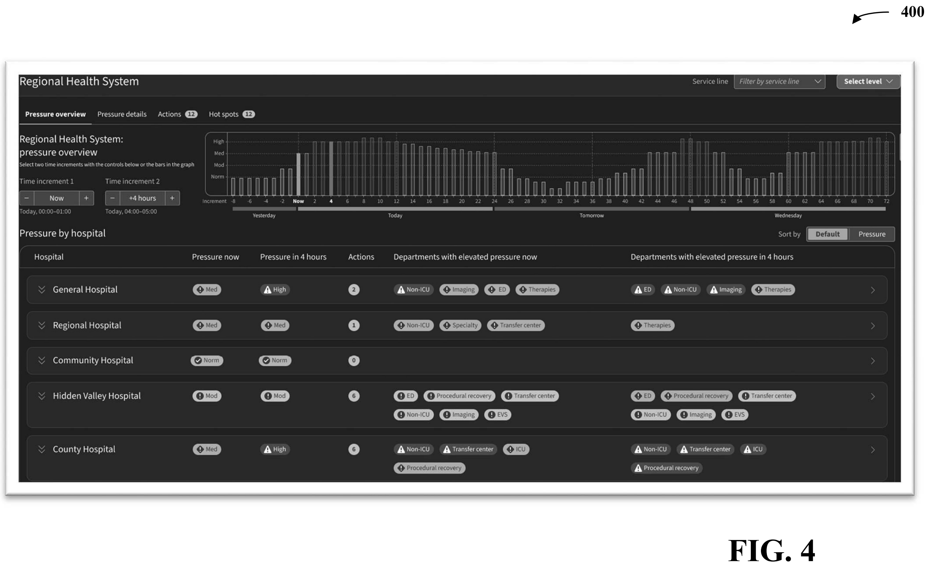

Next, FIG. 4 illustrates an example, non-limiting user interface 400 for visualizing pressure forecasts, current operational pressure levels, and prescriptive actions across multiple healthcare facilities within a regional health system. The interface can provide centralized situational awareness and can be configured to support clinical and operational decision-making at the system, hospital, and department level.

In various embodiments, the top portion of the interface can include a configurable pressure forecast chart that visualizes projected pressure trends over a defined time horizon, such as the next 72 hours. The forecast visualization can be presented as a color-coded bar chart, where each bar represents an hourly or multi-hour pressure projection, and the height and color of the bar reflect the severity of anticipated operational pressure. For example, bars may be shaded green for "Normal" pressure, yellow for "Moderate," orange for "Elevated," and red for "High." This color gradient can be configurable and can allow users to rapidly identify spikes in pressure across time. Users can interact with the time axis by selecting specific increments (e.g., "Now" and "+4 hours") to filter and compare pressure projections across the system.

Below the chart, the interface can display a collapsible, tabular view of pressure data by hospital or facility. Each row can correspond to an individual hospital and can show its name, current pressure level, forecasted pressure in a future time window, the number of associated prescriptive actions, and a list of departments or service lines experiencing elevated pressure. These indicators can be updated in real time and can reflect both nowcasts and forecasts. For instance, a "General Hospital" row may indicate "Medium" pressure currently, but a "High" forecasted pressure in 4 hours, with associated alerts in the Emergency Department, Imaging, and Therapies. Forecasted pressure can be summarized both numerically and visually, using badge-style icons with severity labels (e.g., "Med," "High," "Norm") for easy scanning.

In some implementations, the interface can allow users to drill down into hospital rows to expand and view additional details. These details can include pressure metrics for individual departments (e.g., ICU, Non-ICU, Transfer Center), a list of active or recommended mitigation actions, and links to deeper dashboards or patient flow data. Each department-level pressure tag can be interactive, enabling the user to quickly identify operational hot spots and explore the sources of pressure.

The interface can also support user filtering by service line (e.g., surgery, imaging, procedural services), allowing operations leaders to focus on subsets of the system relevant to their role. Additionally, sorting mechanisms such as "Default" or "Pressure" can reorder the hospital rows based on predefined logic or the severity of current/future pressure.

The user interface 400 can serve as a command center-style visualization that enables healthcare administrators and clinical operations staff to anticipate bottlenecks, evaluate resource constraints, and take proactive steps to mitigate risks. This dashboard-based format can combine AI-driven predictions with clear, actionable displays, empowering healthcare systems to manage pressure holistically across multiple hospitals and service lines.

The visualization style, including color-coded bars, icons, and department badges, can also support rapid cognition and situational triage in high-pressure environments. Forecast horizons, severity thresholds, and prescriptive action tags can all be customizable based on institutional preferences, operational playbooks, or user access levels.

In sum, FIG. 4 demonstrates how complex, multi-facility pressure modeling can be translated into an intuitive, role-sensitive graphical interface that surfaces the right data at the right time to the right stakeholders.

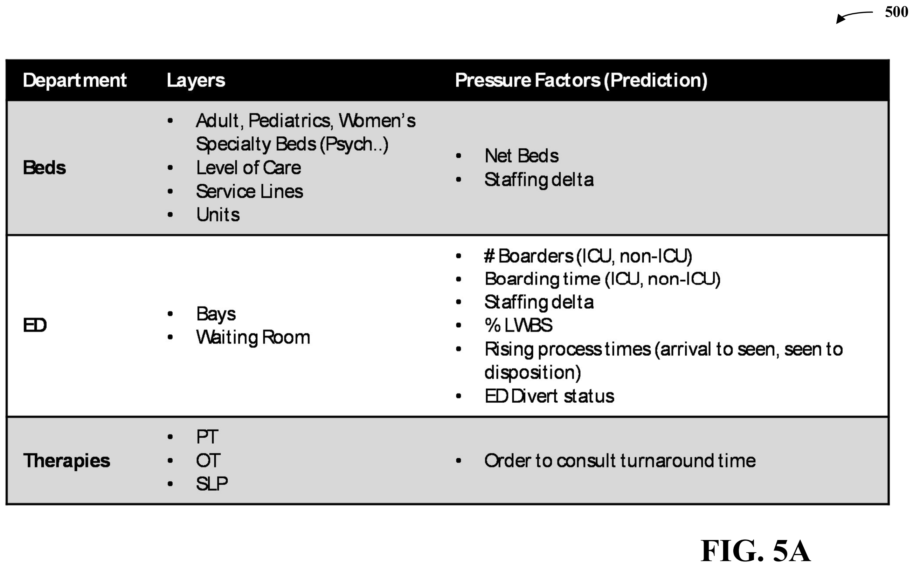

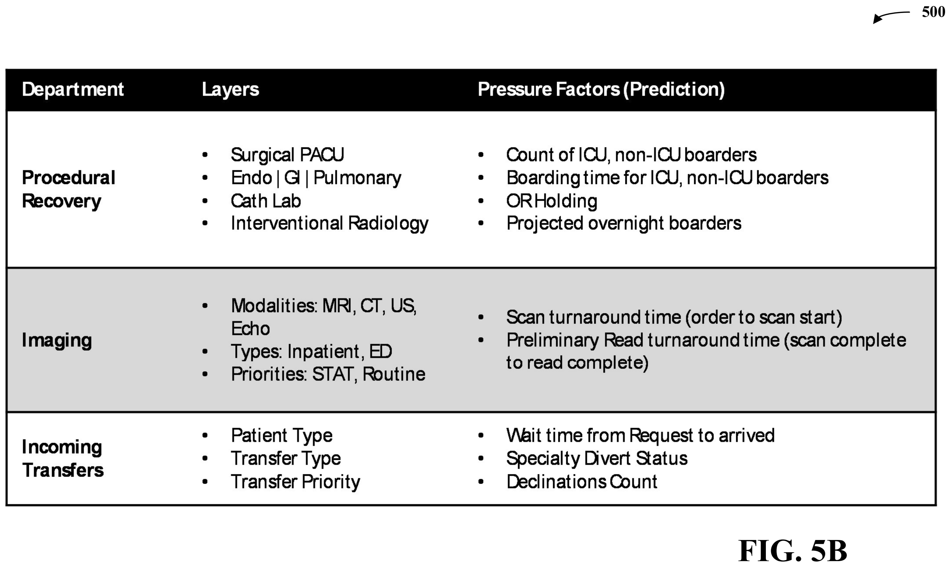

FIGS. 5A and 5B collectively illustrate an example, non-limiting set of department-specific pressure layers and corresponding predictive pressure factors 500, which can be used by a pressure forecasting system to generate department-level pressure scores across a hospital or health system. Each row in FIGS. 5A and 5B identifies a hospital department or functional area, the relevant organizational or service layers within that department, and the predictive features or data elements that can contribute to the department's pressure score.

In FIG. 5A, the first department shown is "Beds," which can be modeled across layers such as adult, pediatrics, women's specialty beds (e.g., psychiatric), level of care (e.g., medical/surgical, telemetry, intensive care), service lines, and specific units. Predictive pressure factors for this department can include the net number of beds available and a calculated staffing delta, representing the deviation from expected or target staffing levels. These variables can be used to assess whether sufficient staffed beds are available to accommodate anticipated patient demand.

The "ED" (emergency department) row in FIG. 5A identifies bays and waiting rooms as relevant sublayers. Pressure in the ED can be driven by several predictive factors, including the number of boarders (patients awaiting inpatient beds) in both ICU and non-ICU areas, average boarding time for each group, staffing delta, the percentage of patients who leave without being seen (LWBS), rising process times (such as time from arrival to provider evaluation or time from provider evaluation to disposition), and the ED's current divert status. These features can provide insight into ED overcrowding, throughput bottlenecks, and care delays.

Also shown in FIG. 5A is the "Therapies" department, which can include physical therapy (PT), occupational therapy (OT), and speech-language pathology (SLP). The primary pressure factor identified for this department is the order-to-consult turnaround time, which reflects the elapsed time between a clinical consult order and the initiation of therapeutic evaluation or treatment. Delays in this metric can signal staffing constraints, inefficiencies in coordination, or excess demand.

FIG. 5B continues the framework with additional departments. The "Procedural Recovery" row includes service layers such as surgical post-anesthesia care units (PACUs), endoscopy, interventional radiology, and the cardiac catheterization lab. Predictive pressure factors for this department can include the number of boarders (ICU and non-ICU), boarding time for those patients, OR holding counts, and the number of projected overnight boarders. These features can indicate capacity strain following procedures, especially when discharge or transfer workflows are delayed.

The "Imaging" department can be modeled across layers including imaging modalities (MRI, CT, ultrasound, echocardiography), patient types (inpatient or ED), and prioritization levels (e.g., STAT vs. routine). Pressure can be predicted based on scan turnaround time (from order entry to scan start) and preliminary read turnaround time (from scan completion to availability of the first interpretation). Long delays in either can reflect imaging backlogs or radiologist capacity limitations.

Finally, the "Incoming Transfers" row reflects workflows related to external patient referrals or system-level transfer coordination. Relevant layers can include patient type (e.g., specialty service, condition severity), transfer type (emergent, urgent, elective), and transfer priority. Predictive pressure features can include wait time from referral to arrival, current specialty divert status, and declination count (i.e., the number of transfers rejected due to lack of capacity or resource mismatch). These features can help assess the system's real-time capacity to absorb external demand.

Together, FIGS. 5A and 5B demonstrate that pressure can be calculated and forecasted using customized predictive variables tailored to the unique operational dynamics of each department. This framework enables pressure modeling at both granular and aggregate levels, supporting system-wide visibility and department-specific mitigation strategies.

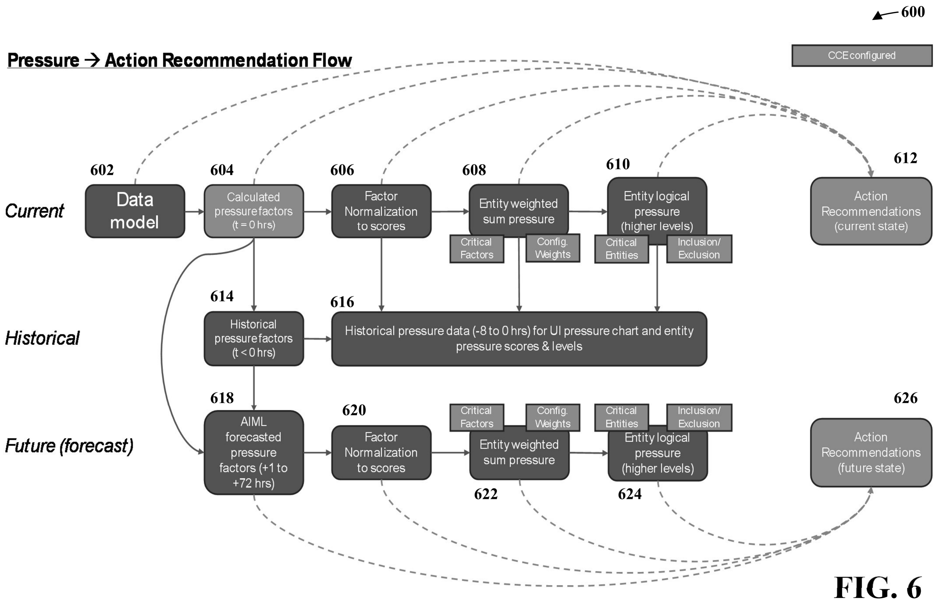

FIG. 6 illustrates an example, non-limiting flow diagram 600 that depicts how operational pressure can be processed and transformed into prescriptive action recommendations using both real-time and forecasted inputs. The process can span three temporal dimensions: current, historical, and future, and can support pressure-driven decision-making at both local and system levels. Each data transformation stage within the flow can be designed to preserve interpretability, traceability, and modularity.

At the leftmost side of the diagram, a data model 602 can collect and organize operational input data from one or more healthcare entities. This data can include real-time feeds of pressure-related metrics from departmental systems, such as imaging turnaround times, staffed bed counts, or procedural backlogs. These inputs can serve as the foundation for calculating pressure factors.

The calculated pressure factors block 604 can represent the immediate processing of current-state data to produce department-specific and domain-specific pressure indicators, such as current boarding times, ED divert status, or order-to-scan turnaround times. These pressure factors can be normalized into standardized scores in block 606 using statistical transforms or percentile-based mappings, enabling consistent interpretation across departments and over time.

In block 608, the normalized scores can be aggregated into an entity-weighted pressure score. This step can compute a composite pressure score for a department, unit, or hospital entity by applying configuration weights to each pressure factor. For example, a procedural recovery unit's score may emphasize discharge delays more heavily than staffing counts, depending on operational priorities.

The resulting scores can be used to determine entity-level logical pressure classifications in block 610, which can be computed using proportional thresholds and override logic. These classifications can include labels such as Normal, Moderate, Elevated, or High and may be assigned at multiple organizational levels, for example at the unit, service line, or facility level. In some embodiments, the inclusion or exclusion of certain sub-entities or data elements can be configured dynamically based on operational rules or seasonal variation.

Once the current-state pressure levels are finalized, the system can generate action recommendations in block 612 designed to mitigate or respond to detected pressure conditions. These recommendations can take into account the criticality of contributing factors, the feasibility of actions, and potential downstream impacts. Examples can include reassigning float staff, expediting consult orders, or activating escalation workflows.

In parallel, the system can track and store historical pressure factor values in block 614, which can be used to build an eight-hour trailing data window represented in block 616 for trend visualization and context awareness. This historical data can feed downstream forecasting models and contribute to pressure stability calculations, for example for hysteresis smoothing.

Forecasted pressure processing can begin at block 618, where one or more artificial intelligence or machine learning models can generate future pressure factor estimates using trends observed in historical inputs. These forecasts can span from one hour to seventy-two hours in the future and can be continuously updated as new data is received.

The predicted pressure factors can be normalized to pressure scores in block 620 and processed in the same manner as the current-state logic. In block 622, weighted scores can be calculated for each forecasted entity, and in block 624, logical pressure classifications can be assigned using proportional and override techniques. These outputs can then feed into future-state action recommendations in block 626, allowing clinical or operational teams to plan interventions ahead of anticipated constraints or bottlenecks.

Throughout the flow, each processing path, including current, historical, and future, can contribute data to shared visualizations, reports, or dashboards. Dashed arrows in FIG. 6 can represent information feedback loops that support reforecasting, cross-validation, or refinement of pressure classifications. All transformation stages can be logged and made auditable, ensuring that both the raw data and the resulting recommendations are explainable and reproducible. The entire flow depicted in FIG. 6 can support pressure-aware decision-making across operational domains and time horizons, enabling both reactive and proactive hospital management.

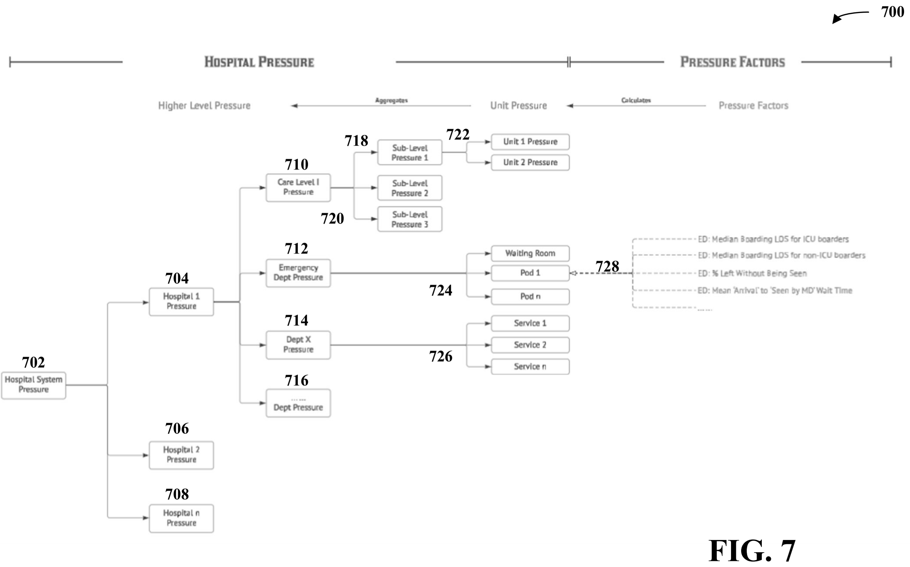

FIG. 7 illustrates a hierarchical pressure aggregation model 700 that can represent how operational pressure scores can be derived and rolled up from granular pressure factors to higher-level hospital system pressure indicators. This model can support multi-layer pressure modeling and visualization across units, departments, hospitals, and systems.

The hierarchy can begin at the system level with a hospital system pressure score, represented at block 702. This pressure score can reflect the overall operational burden across all included facilities within a health system. The hospital system pressure score can be derived from aggregating individual pressure scores for each hospital, such as Hospital 1 at block 704, Hospital 2 at block 706, and Hospital n at block 708. These individual hospital pressure scores can be based on multiple department and unit scores calculated within each respective facility.

For example, Hospital 1 pressure at block 704 can be generated by aggregating multiple departmental and care-level pressures, including Care Level I pressure at block 710, Emergency Department (ED) pressure at block 712, and one or more other department-specific pressures at blocks 714 and 716. Each department or care level can reflect a separate clinical or operational function within the hospital and can independently contribute to overall pressure.

Within Care Level I pressure at block 710, additional sub-level pressures can be modeled. As shown at block 718, these can include Sub-Level Pressure 1, Sub-Level Pressure 2, and Sub-Level Pressure 3, each reflecting operational pressure within distinct divisions or classifications, such as by patient acuity or service type. These sub-level pressures can further feed into specific unit-level pressures, such as Unit 1 Pressure and Unit 2 Pressure, shown at block 722. These unit pressures can represent the most granular layer in the hierarchy, often corresponding to actual physical spaces or clinical units such as medical wards or intensive care units.

The Emergency Department pressure at block 712 can be similarly decomposed into individual components. Block 724 shows example unit layers such as a Waiting Room, Pod 1, and additional Pods, each of which can be treated as independently monitored zones within the emergency department. These zones can calculate local pressure values that roll up into the overall ED pressure score. Additional services such as Service 1, Service 2, and Service n are shown at block 726 to illustrate how modular service lines or support functions can also contribute discrete pressure values.

At the most granular level, pressure values can be generated based on operational metrics and real-time signals. For instance, Pod 1 within the emergency department can monitor a set of defined pressure factors, depicted at block 728. These can include metrics such as the median boarding length of stay (LOS) for ICU boarders, the median boarding LOS for non-ICU boarders, the percentage of patients who left without being seen (LWBS), and the average time from arrival to being seen by a physician. These factors can be normalized, weighted, and aggregated to generate a composite pressure score for the unit or pod.

Each upstream layer can then compute a weighted sum or logical aggregation of its constituent units' pressure scores, enabling progressive roll-up from pressure factors to unit pressure, to department pressure, to hospital-level pressure, and finally to system-level pressure. This structure can support traceability and interpretability by allowing users to drill down from a high-level pressure indicator to its underlying contributors.

The illustrated framework in FIG. 7 can enable hospital administrators and operational teams to detect bottlenecks, forecast system stress, and coordinate cross-departmental interventions. The hierarchical model can also support real-time dashboarding, visual heatmaps, or escalation workflows tied to pressure thresholds at any level of the hierarchy.

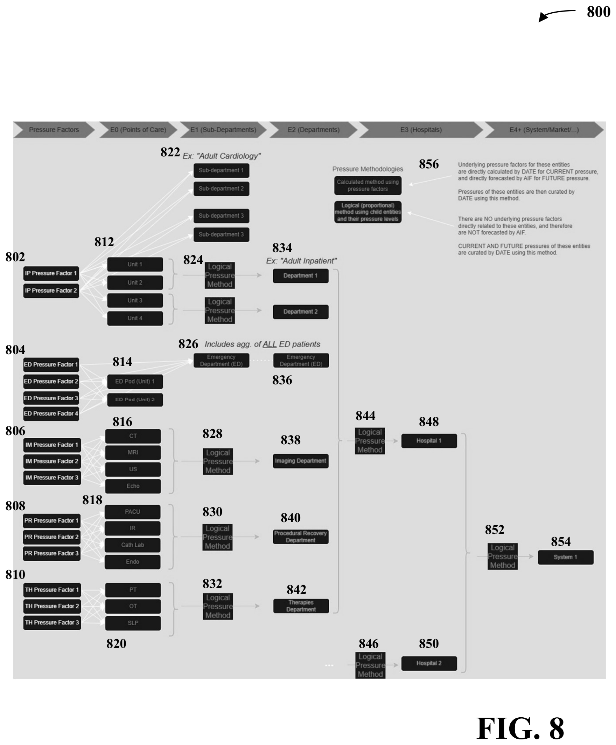

Next, FIG. 8 illustrates an example hierarchical framework 800 for calculating, aggregating, and interpreting pressure within a healthcare network. This multi-layered pressure modeling structure can facilitate the classification and propagation of pressure signals across multiple organizational levels, ranging from individual care points to system-level rollups. The pressure factors, units, departments, hospitals, and systems illustrated in the figure can be used to support real-time pressure scoring, visualization, and prescriptive action recommendations across clinical and operational contexts.

Starting at the bottom left of the diagram, a set of domain-specific pressure factors can be defined for each clinical domain. For example, blocks 802, 804, 806, 808, and 810 illustrate pressure factors associated with various care settings, such as inpatient units (IP), emergency departments (ED), imaging modalities (IM), procedural recovery (PR), and therapy departments (TH). These pressure factors can include real-time metrics such as staffing deltas, boarding times, scan turnaround times, and patient throughput indicators. These values can be calculated continuously or at fixed intervals and used as inputs into pressure scoring routines.

Above each set of pressure factors, points of care such as units or modalities can be defined. As shown in block 812, the IP domain can comprise multiple units (e.g., Unit 1, Unit 2, Unit 3, Unit 4), each of which can be scored independently based on its corresponding pressure factors. Similarly, blocks 814 through 820 illustrate the propagation of ED pressure factors to ED pods, imaging pressure factors to modalities (CT, MRI, US, Echo), procedural recovery pressure factors to service areas (PACU, IR, Cath Lab, Endo), and therapy pressure factors to service lines (PT, OT, SLP).

Pressure values for each of these lower-level entities can be normalized and then passed through a logical pressure aggregation method, as indicated by blocks 824, 826, 828, 830, and 832. This logical method can proportionally combine sub-entity pressure values using configurable weights, inclusion rules, or criticality thresholds. The outputs of these aggregations can serve as department-level pressure indicators.

Departments are shown at the E2 level, as seen in blocks 834 through 842. These departments can include groupings such as Adult Inpatient, Emergency Department, Imaging, Procedural Recovery, and Therapies. Each department can receive pressure values from its constituent sub-units and propagate that pressure upward if thresholds are exceeded or if critical services are affected. For example, if CT and MRI both experience high pressure, the Imaging Department as a whole may be classified as under elevated pressure.

Department-level pressures can then be aggregated at the hospital level. As shown in blocks 844 through 850, the logical pressure method can be used again to compute overall hospital pressure scores. These scores can reflect current operational stress across the institution and can incorporate pressure signals from multiple domains simultaneously. For instance, elevated pressure in ED, Imaging, and Procedural Recovery can all contribute to Hospital 1's total pressure score.

At the top of the hierarchy, block 854 illustrates how multiple hospitals (e.g., Hospital 1, Hospital 2) can roll up into a system-level pressure indicator. Block 852 shows that this system-level score can be derived using the same logical pressure method applied at prior levels, allowing consistent interpretation across the entire enterprise. The system-level score can be useful for health system leadership, regional coordinators, or state-level administrators when managing capacity and resource allocation across hospitals.

Finally, the figure distinguishes between calculated pressure methodologies and logical aggregation methods, as shown in explanatory block 856. Calculated methods can apply when pressure factors exist for a given entity and can be used to forecast pressure based on AI or statistical models. Logical aggregation methods can apply when pressure is derived from underlying child entities that do have direct pressure inputs. For example, a procedural recovery department may use calculated methods, whereas a system-level pressure score may use logical rollups.

This hierarchical approach can allow for pressure tracking and escalation across multiple levels of granularity and can support both current-state assessments and future-state forecasting. The structure shown in FIG. 8 can be implemented to provide contextual awareness, trigger escalation workflows, and enable proactive system management in complex care environments.

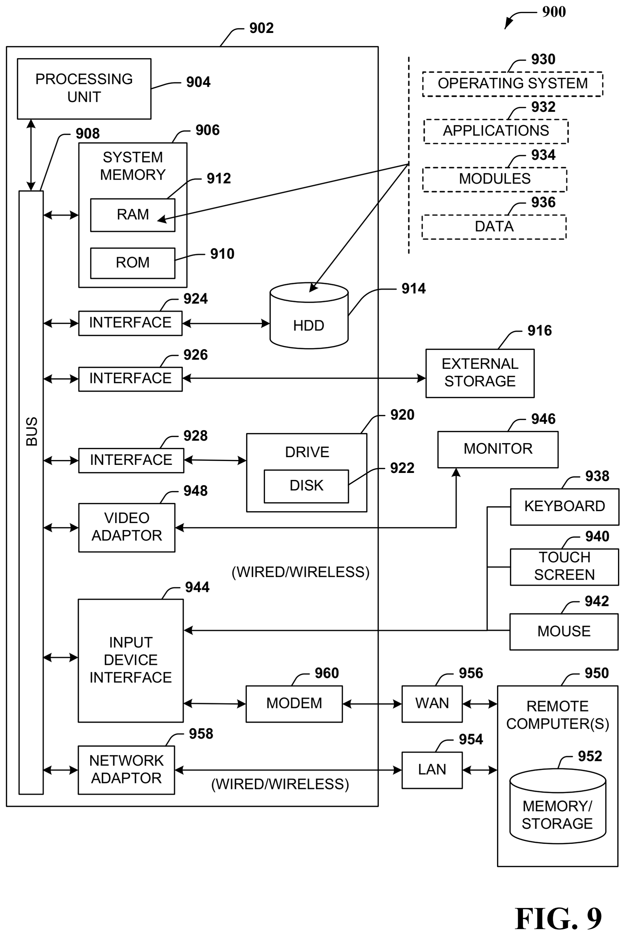

In order to provide additional context for various embodiments described herein, FIG. 9 and the following discussion are intended to provide a brief, general description of a suitable computing environment 900 in which the various embodiments of the embodiment described herein can be implemented. While the embodiments have been described above in the general context of computer-executable instructions that can run on one or more computers, those skilled in the art will recognize that the embodiments can also be implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 9, the example environment 900 for implementing various embodiments of the aspects described herein includes a computer 902, the computer 902 including a processing unit 904, a system memory 906 and a system bus 908. The system bus 908 couples system components including, but not limited to, the system memory 906 to the processing unit 904. The processing unit 904 can be any of various commercially available processors. Dual microprocessors and other multi-processor architectures can also be employed as the processing unit 904.

The system bus 908 can be any of several types of bus structure that can further interconnect to a memory bus (with or without a memory controller), a peripheral bus, and a local bus using any of a variety of commercially available bus architectures. The system memory 906 includes ROM 910 and RAM 912. A basic input/output system (BIOS) can be stored in a non-volatile memory such as ROM, erasable programmable read only memory (EPROM), EEPROM, which BIOS contains the basic routines that help to transfer information between elements within the computer 902, such as during startup. The RAM 1912 can also include a high-speed RAM such as static RAM for caching data.

The computer 902 further includes an internal hard disk drive (HDD) 914 (e.g., EIDE, SATA), one or more external storage devices 916 (e.g., a magnetic floppy disk drive (FDD) 916, a memory stick or flash drive reader, a memory card reader, etc.) and a drive 920, e.g., such as a solid state drive, an optical disk drive, which can read or write from a disk 922, such as a CD-ROM disc, a DVD, a BD, etc. Alternatively, where a solid state drive is involved, disk 922 would not be included, unless separate. While the internal HDD 914 is illustrated as located within the computer 902, the internal HDD 914 can also be configured for external use in a suitable chassis (not shown). Additionally, while not shown in environment 900, a solid state drive (SSD) could be used in addition to, or in place of, an HDD 914. The HDD 914, external storage device(s) 916 and drive 920 can be connected to the system bus 908 by an HDD interface 924, an external storage interface 926 and a drive interface 928, respectively. The interface 924 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 902, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 912, including an operating system 930, one or more application programs 932, other program modules 934 and program data 936. All or portions of the operating system, applications, modules, or data can also be cached in the RAM 912. The systems and methods described herein can be implemented utilizing various commercially available operating systems or combinations of operating systems.

Computer 902 can optionally comprise emulation technologies. For example, a hypervisor (not shown) or other intermediary can emulate a hardware environment for operating system 930, and the emulated hardware can optionally be different from the hardware illustrated in FIG. 9. In such an embodiment, operating system 930 can comprise one virtual machine (VM) of multiple VMs hosted at computer 902. Furthermore, operating system 930 can provide runtime environments, such as the Java runtime environment or the .NET framework, for applications 932. Runtime environments are consistent execution environments that allow applications 932 to run on any operating system that includes the runtime environment. Similarly, operating system 930 can support containers, and applications 932 can be in the form of containers, which are lightweight, standalone, executable packages of software that include, e.g., code, runtime, system tools, system libraries and settings for an application.

Further, computer 902 can be enabled with a security module, such as a trusted processing module (TPM). For instance, with a TPM, boot components hash next in time boot components, and wait for a match of results to secured values, before loading a next boot component. This process can take place at any layer in the code execution stack of computer 902, e.g., applied at the application execution level or at the operating system (OS) kernel level, thereby enabling security at any level of code execution.

A user can enter commands and information into the computer 902 through one or more wired/wireless input devices, e.g., a keyboard 938, a touch screen 940, and a pointing device, such as a mouse 942. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 904 through an input device interface 944 that can be coupled to the system bus 908, but can be connected by other interfaces, such as a parallel port, an IEEE 1394 serial port, a game port, a USB port, an IR interface, a BLUETOOTH^{®} interface, etc.

A monitor 946 or other type of display device can also be connected to the system bus 908 via an interface, such as a video adapter 948. In addition to the monitor 946, a computer typically includes other peripheral output devices (not shown), such as speakers, printers, etc.

The computer 902 can operate in a networked environment using logical connections via wired or wireless communications to one or more remote computers, such as a remote computer(s) 950. The remote computer(s) 950 can be a workstation, a server computer, a router, a personal computer, portable computer, microprocessor-based entertainment appliance, a peer device or other common network node, and typically includes many or all of the elements described relative to the computer 902, although, for purposes of brevity, only a memory/storage device 952 is illustrated. The logical connections depicted include wired/wireless connectivity to a local area network (LAN) 954 or larger networks, e.g., a wide area network (WAN) 956. Such LAN and WAN networking environments are commonplace in offices and companies, and facilitate enterprise-wide computer networks, such as intranets, all of which can connect to a global communications network, e.g., the Internet.

When used in a LAN networking environment, the computer 902 can be connected to the local network 954 through a wired or wireless communication network interface or adapter 958. The adapter 958 can facilitate wired or wireless communication to the LAN 954, which can also include a wireless access point (AP) disposed thereon for communicating with the adapter 958 in a wireless mode.

When used in a WAN networking environment, the computer 902 can include a modem 960 or can be connected to a communications server on the WAN 956 via other means for establishing communications over the WAN 956, such as by way of the Internet. The modem 960, which can be internal or external and a wired or wireless device, can be connected to the system bus 908 via the input device interface 944. In a networked environment, program modules depicted relative to the computer 902 or portions thereof, can be stored in the remote memory/storage device 952. It will be appreciated that the network connections shown are example and other means of establishing a communications link between the computers can be used.

When used in either a LAN or WAN networking environment, the computer 902 can access cloud storage systems or other network-based storage systems in addition to, or in place of, external storage devices 916 as described above, such as but not limited to a network virtual machine providing one or more aspects of storage or processing of information. Generally, a connection between the computer 902 and a cloud storage system can be established over a LAN 954 or WAN 956 e.g., by the adapter 958 or modem 960, respectively. Upon connecting the computer 902 to an associated cloud storage system, the external storage interface 926 can, with the aid of the adapter 958 or modem 960, manage storage provided by the cloud storage system as it would other types of external storage. For instance, the external storage interface 926 can be configured to provide access to cloud storage sources as if those sources were physically connected to the computer 902.

The computer 902 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

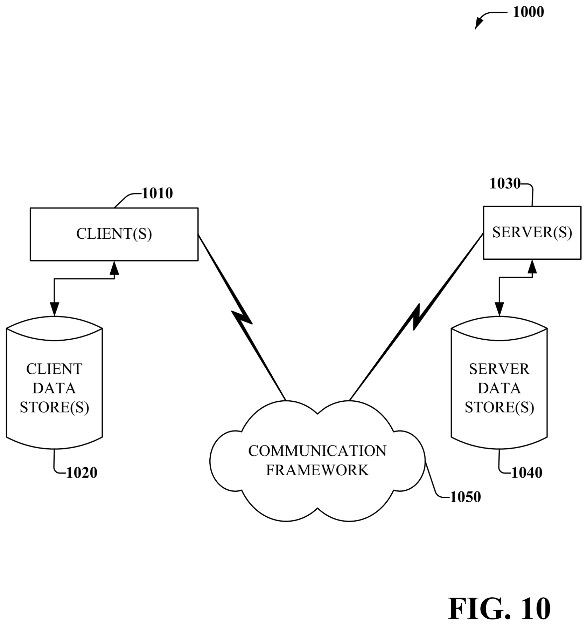

FIG. 10 is a schematic block diagram of a sample computing environment 1000 with which the disclosed subject matter can interact. The sample computing environment 1000 includes one or more client(s) 1010. The client(s) 1010 can be hardware or software (e.g., threads, processes, computing devices). The sample computing environment 1000 also includes one or more server(s) 1030. The server(s) 1030 can also be hardware or software (e.g., threads, processes, computing devices). The servers 1030 can house threads to perform transformations by employing one or more embodiments as described herein, for example. One possible communication between a client 1010 and a server 1030 can be in the form of a data packet adapted to be transmitted between two or more computer processes. The sample computing environment 1000 includes a communication framework 1050 that can be employed to facilitate communications between the client(s) 1010 and the server(s) 1030. The client(s) 1010 are operably connected to one or more client data store(s) 1020 that can be employed to store information local to the client(s) 1010. Similarly, the server(s) 1030 are operably connected to one or more server data store(s) 1040 that can be employed to store information local to the servers 1130.

Various embodiments can be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The disclosure herein describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system, comprising:
a processor that executes computer-executable components stored in a non-transitory computer-readable memory, wherein the computer-executable components comprise:
a data ingestion component that receives operational data associated with a plurality of healthcare entities within a hospital system;
a pressure modeling component that calculates real-time pressure scores for the healthcare entities based on department-specific pressure factors and weightings;
a forecasting component that applies an artificial intelligence model to forecast pressure scores for the healthcare entities over a future time window;
a scoring component that assigns pressure levels to healthcare entities using proportional or override logic based on critical factors or critical entities;
an action recommendation component that identifies one or more prescriptive actions for reducing or preventing forecasted pressure, each action associated with an impact score, a cost score, and a trigger condition; and
a user interface component that displays a graphical interface comprising a pressure forecast visualization, a dynamic hot spot summary, and one or more selectable actions.

2. The system of claim 1, wherein the pressure modeling component normalizes pressure factor values into percentile-based scores.

3. The system of claim 1, wherein the forecasting component generates hourly pressure score predictions for each eligible healthcare entity across a forecast time horizon.

4. The system of claim 1, wherein the scoring component propagates pressure levels from subordinate to parent entities.

5. The system of claim 1, wherein the scoring component overrides pressure scores based on a critical factor exceeding a configured threshold.

6. The system of claim 1, wherein the action recommendation component ranks the prescriptive actions based on a composite of the impact score and the cost score.

7. The system of claim 1, wherein the user interface component displays multiple hot spots at a time, each comprising a summary indicating one or more drivers of pressure.

8. The system of claim 1, wherein the user interface component includes a selectable icon that launches a chat agent for scenario analysis based on forecasted pressure.

9. The system of claim 1, wherein the selectable actions include actions to clean beds, initiate transport, reassign staff, or downgrade care levels.

10. A computer-implemented method, comprising:
receiving operational data from a plurality of healthcare entities within a hospital system;
calculating pressure scores for the healthcare entities based on a weighted sum of normalized pressure factor values;
forecasting future pressure scores using an artificial intelligence model trained on historical pressure data;
assigning pressure levels to healthcare entities using override logic for critical factors and proportional logic for aggregate entities;
identifying prescriptive actions to address current or forecasted pressure, each action having a trigger condition, impact score, and cost score; and
displaying, via a graphical user interface, a pressure forecast visualization, a hot spot summary, and selectable recommended actions.

11. The method of claim 10, further comprising updating forecasted pressure scores at a predetermined time interval and storing historical pressure data.

12. The method of claim 10, wherein the assigning of pressure levels includes categorizing pressure as Normal, Moderate, Elevated, or High based on pressure scores.

13. The method of claim 10, further comprising generating a ranked list of actions based on priority level and impact-to-cost ratio.

14. The method of claim 10, further comprising enabling a user to click through an action to view linked patient lists, bed availability, or staffing resources.

15. A computer program product for forecasting and managing operational pressure in a healthcare system, the computer program product comprising a non-transitory computer-readable memory having program instructions embodied therewith, the program instructions executable by a processor to:
receive operational data from a plurality of healthcare entities;
generate real-time pressure scores based on department-specific pressure factor scores and weightings;
forecast future pressure conditions for each entity using an artificial intelligence model trained on historical data;
assign pressure levels to each entity using override and proportional logic;
identify prescriptive actions with associated trigger conditions, impact scores, and cost scores; and
display pressure forecasts, hot spots, and selectable action recommendations within a graphical user interface.
